# EUROPEAN PATENT APPLICATION

(11) **EP 1 138 682 A1**
(43) Date of publication of application: **04.10.2001**
(21) Application number: 01115319.4
(22) Date of filing: 22.04.1997
(51) Int. Cl.: C07D 491/22, A61K 31/47, A61P 35/00

(54) **Novel water soluble c-ring analogues of 20 (s)-camptothecin**

(30) Priority: 19.12.1996 US 771391
(62) Divisional of application: 97936012.0
(71) Applicant: DR. REDDY'S RESEARCH FOUNDATION, Hyderabad, 500 016 Andra Pradesh (IN); Reddy-Cheminor, Inc., Ridgewood, NJ 07450 (US)
(72) Inventor: Duvvuri, Subrahmanyam, Hyderabad-500, 050 Andhra Pradesh (IN); Akella, Venkateswarlu, Hyderabad-500, 050 Andhra Pradesh (IN); Vedula, Sharma Manohara, Hyderabad-500, 050 Andhra Pradesh (IN); Madabhushi, Shobha, Hyderabad-500, 050 Andhra Pradesh (IN); Chintakunta, Vamsse Krishna, Hyderabad-500, 050 Andhra Pradesh (IN); Thungathurthi, Sastry V.R.S., Hyderabad-500, 050 Andhra Pradesh (IN)
(74) Representative: Ebner von Eschenbach, Jennifer

(57) **Abstract**

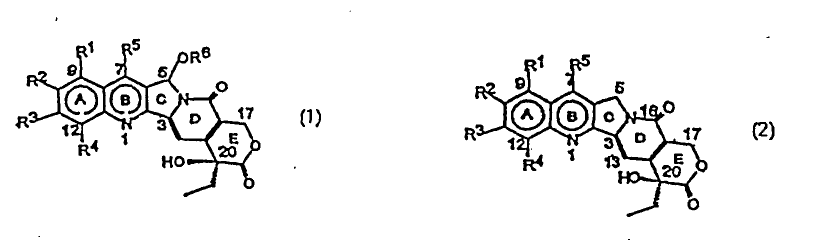

Novel water-soluble C-ring analogues of 20(s)-camptothecin having general formula (1). All the compounds of formula (1) are prepared from the compounds of general formula (2) having 20(s)-chiral carbon where R¹ to R⁵ have the meaning given above. The compounds of formula (1) possess potent anti-cancer and anti-viral properties. The invention also provides an alternate process for the preparation of known C-5 substituted compounds of formula (1).

## Description

The present invention relates to novel water soluble C-ring analogues of 20(S)-Camptothecin having the general formula 1.

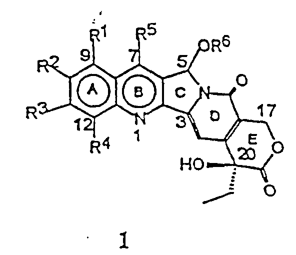

In the above formula 1, R¹, R², R³, R⁴ are independently the same or different and represent hydrogen, hydroxy, aryloxy, lower alkoxy, lower alkanoyl, nitro, cyano, halo, carboxy, carbonyloxy, amino, substituted amino, lower alkyl, substituted lower alkyl, or R², R³ together represent -O-(CH₂)ₙ-O- where n=1 or 2; R⁵ represents hydrogen, lower alkyl, substituted lower alkyl, lower aralkyl, hydroxymethyl, carboxymethyl, aminomethyl, substituted aminomethyl where the amino group may be mono or disubstituted in which both substituents are independent or combined together to form a cyclic ring system of a total of 5-6 atoms containing carbon and optionally one or two heteroatoms selected from oxygen, nitrogen or sulfur; and R⁶ represents hydrogen, phenyl or benzyl where the phenyl group may be unsubstituted or substituted with mono, di or trisubstituents which may be selected from halogen, hydroxy, lower alkoxy, cyano, carboxyl, nitro, amino or substituted amino, lower alkyl, substituted lower alkyl; cycloalkyl or cycloalkyl lower alkyl where the cyclic ring is in the range of 3 membered to 7 membered ring system containing all carbon atoms; lower alkyl groups substituted with heterocyclic rings where the heterocyclic ring system has a total of 3 to 7 atoms, the heterocyclic rings containing carbon with at least one heteroatom such as oxygen, nitrogen or sulfur; lower alkanoyl; benzoyl where the phenyl group can be unsubstituted or substituted; lower alkenyl; lower alkyl; substituted lower alkyl, substituted lower alkenyl or substituted lower alkanoyl where the substituents can be halogen, hydroxy, lower alkoxy, aryloxy, thio, thioalkyl, thioaryl, aryl, heteroaryl, carboxy, cyano, nitro, amido or amino in which the amino group can be unsubstituted or mono, or disubstituted in which both substituents are independent or combined together to form 5 or 6 membered cyclic ring system containing carbon, and optionally contain one or two heteroatoms selected from oxygen, nitrogen or sulfur, the total number of atoms in the cyclic ring system is 5 or 6; with the proviso that (i) when R¹ is methoxy group, R⁶ is not hydrogen or lower alkyl group; (ii) when R² is hydroxy, lower alkoxy, thioalkyl, nitro, amino, alkylamino, acylamino or halogen, R⁶ is not hydrogen or lower alkyl group; (iii) when R⁵ is lower alkyl, lower aralkyl, CH₂OH, COOH, COOMe or CH₂OR" where R" represents lower alkyl or acyl group, R⁶ is not hydrogen or lower alkyl group; (iv) when R¹ is methoxy group, R² is hydroxy, lower alkoxy, thioalkyl, nitro, amino, alkylamino, acylamino, or halogen, R⁵ is lower alkyl, lower aralkyl, CH₂OH, COOH, COOMe or CH₂OR" where R" represents lower alkyl or acyl group, R⁶ is not hydrogen or lower alkyl group; (v) when R¹ through R⁵ represent hydrogen, R⁶ is not hydrogen or lower alkyl group.

All these compounds of the formula 1 are prepared from the compounds of the general formula 2 having 20(S)-chiral center, 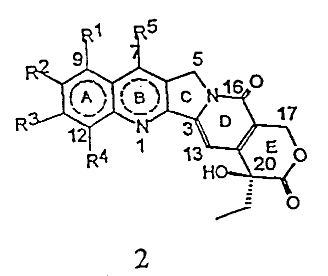 where R¹ to R⁵ have the meaning described above. Camptothecin having the formula 3, is an alkaloid with strong antitumor activity, 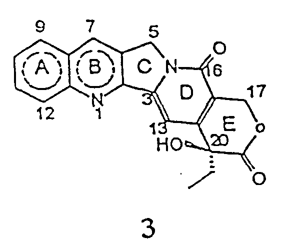 and was isolated from Camptotheca acuminata by Wall and co-workers in 1966. However, its development as a potential drug for cancer treatment had been abandoned due to unacceptable side effects on humans and due to its low water solubility as well as high toxicity problems. Since the discovery of its mechanism of action as an inhibitor of topoisomerise I by Liu and co-workers in 1985 [L.F. Liu, et al., *J*. *Biol*. *Chem.,* 260, 14873 (1985)], the research interest on camptothecin has once again taken momentum.

To overcome this problem of low water solubility and high toxicity of camptothecin, over the last 30 years, several research groups all over the world have prepared and investigated a number of camptothecin analogues involving the modification of rings A-E or the introduction of a variety of substituents on all the five rings of camptothecin of the formula 3 [M. E. Wall et al., *J. Med. Chem*., 36, 2689 (1993); R. P. Hertzberg et al., *J. Med. Chem.*, 715 (1989); S. W. Sawada et al., Chem. *Pharm. Bull.*, 41(2), 310 (1993)]. Among the various camptothecin analogues prepared to date, only two of them namely, CPT-11 having the formula 4 [*Chem. Pharm. Bull.,* 39, 1446 (1991)], 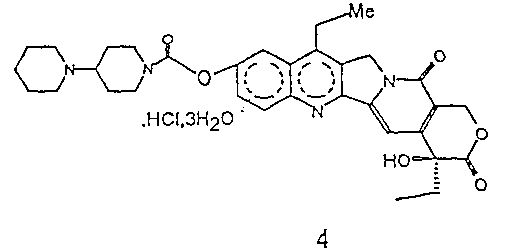 topotecan of the formula 5 [*J. Med. Chem.*, 34, 98(1991)] 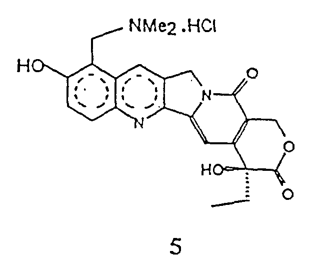 were introduced as anti-cancer drugs in the market recently. Another compound namely, 9-aminocamptothecin of the formula 6 [*J*. *Med. Chem.,* 29, 2358 (1986)], 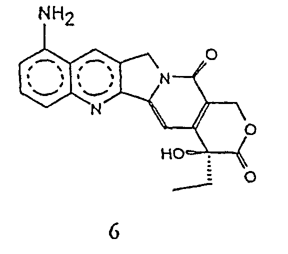 is currently undergoing extensive clinical trials. The extensively studied Structure Activity Relationship (SAR) on camptothecin of the formula 3 [M. E. Wall et al., *J. Med. Chem.,* 36, 2689 (1993)] has revealed that 20(S)-∝-hydroxy-δ-lactone (E-ring) moiety in camptothecin is essential for its activity. However, according to recent reports by Ejima et al., replacement of hydroxyl group with an amino group at C-20 position leading to a compound such as 7-ethyl-10-methoxycamptothecin derivative of the formula 7 [A. Ejima et al., *Chem. Pharm. Bull.,* 40(3), 683 (1992)], 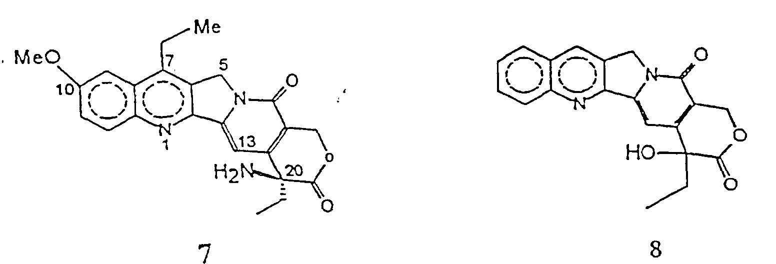 exhibited an increased in vivo antitumor activity than 20(RS)-camptothecin of the formula 8. Also in another report (Lawrence Snyder et al., *J. Org. Chem.*, 59, 7033 (1994)], the 18-noranhydrocamptothecin analogue of the formula 9, 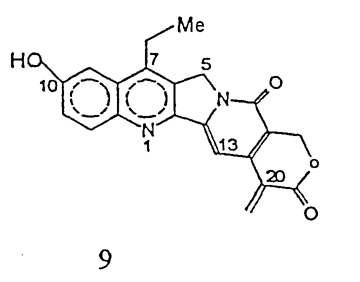 exhibited potent camptothecin like inhibition of topoisomerase I activity. Both these reports are contrary to the assumption that 20(S)-∝-hydroxy functionality in camptothecin is an essential feature for its biological activity.

Based on the structure-activity results obtained for the camptothecin analogues prepared in the literature, it was established that the modification of substituents at C-9 and C-7 position of camptothecin of the formula 3 plays an important role in the enhancement of anticancer activity by imparting stability to the E-ring lactone [T. G. Burke et al., *J. Med. Chem.* 37, 40 (1994)]. It has also been recognized that the open form of the lactone moiety, namely, 'the *Carboxylate form'* is less effective therapeutically than the closed 'Lactone *form'* [Hertzberg et al., *J. Med. Chem.*, 32, 715(1989); J. M. Covey, C. Jaxel et al., *Cancer Research.*, 49, 5016 (1989); Giovanella et al., *Cancer Research.*, 51, 3052 (1991)]. The recent studies by T. G. Burke et al., on the stability of '*closed lactone form*' of various camptothecin analogues in the presence of protein called 'Human Serum Albumin' (HSA) indicated that the compounds such as CPT-11 of the formula 4 and 7-ethyl-10-hydroxycamptothecin (SN-38) of the formula 7a 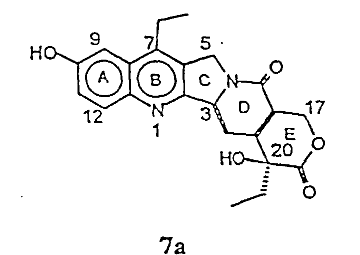 and Topotecan of the formula 5, in the presence of HSA at 37°C., exhibited a higher percentage (%) of lactone form at equilibrium than 20(S) camptothecin of the formula 3 and 9-aminocamptothecin of the formula 6 [T. G. Burke and Zihou Mi., *J. Med. Chem*., 37, 40 (1994); ibid., *Biochemistry*., 33, 12540 (1994).]. Based on these studies, it was recognized that the understanding of the factors influencing the lactone-carboxylate equilibrium of camptothecin analogues became an important determinant in the design of novel and therapeutically efficacious drug candidates in the camptothecin series.

Although the modification of substituents on rings A and B of camptothecin was taken up at a rapid pace to generate novel CPT analogues, ring 'C' analogues of camptothecins were limited presumably because of the research work carried out by Sawada et al., which claimed that the substituents at C-5 position of camptothecin has resulted in the reduction of anti-tumor activity of camptothecins and produced inactive analogues [Sawada *S*. et al., *Chem. Pharm. Bull.,* 39(10), 2574 (1991)]. The C-5 substituted camptothecins claimed by Sawada et al., (JP 58,154,584; US 4,513,138; US 4,473,692; US 4,545,880; US 4,339,282) have the structural formula 10, 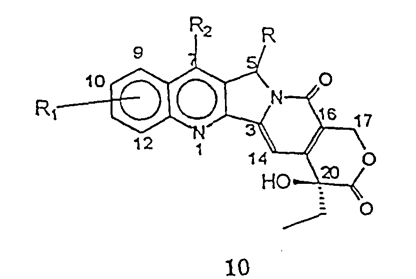 where R represents hydroxy, lower alkyl, lower alkoxy, acyloxy groups, R¹ represents hydrogen, methoxy at 9th position; hydrogen, hydroxy, lower alkoxy, acyloxy, SH, thioalkyl, thioacyl, nitro, amino, alkylamino, acylamino and halogen groups at 10th position and R² represents hydrogen, lower alkyl, lower aralkyl, CH₂OH, COOH, COOMe, CH₂OR' where R' represents lower alkyl or acyl group.

The recent findings by K. H. Lee et al., [*Bio*. *Org. Med. Chem*. *Lett.,* 5(1), 77 (1995)] which includes the preparation of 5-hydroxymethyl camptothecin by the reaction of formaldehyde in N,N-dimethylformamide and 4-piperidinopiperidine on 20(S)-camptothecin, has revealed the reduced anti-tumor activity of these compounds. Also, Danishefsky et al., prepared some of the C-5 substituted 20(RS)-camptothecin derivatives by a totally synthetic approach [US 5,391,745 and US 5,446,047].

However, the synthetically prepared 5-substituted camptothecin derivative of the formula 11 [Terasawa et al., *Heterocycles,* 38, 81 (1994)] claimed to have anti-tumor activity comparable to that of 20(S)-camptothecin.

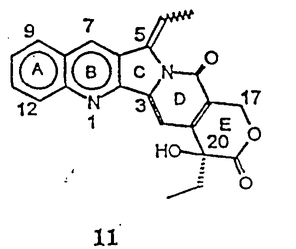

Keeping all these factors in mind, we focused our research studies on 20(S)-camptothecin aimed at the design of novel camptothecin analogues which can exhibit improved water solubility and improved stability of lactone form in solution. We identified a oxidative reaction in alcoholic solvents for this purpose. The resultant findings have culminated into the discovery of a novel synthetic transformation which can introduce a variety of alkoxy groups at C-5 position of 20(S)-camptothecins. Functional group transformation of these 5-alkoxy camptothecins produced a wide variety of novel C-5 substituted 20(S)-camptothecin analogues of the formula 14, 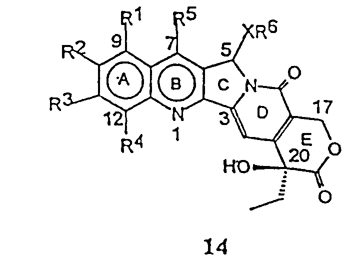 in which X represents NH or NR and CH₂ or CHR and R⁶ has the meaning described above, which is the subject matter of our co-pending application for patents bearing U.S. application serial number 08/771,390 now U.S. Patent 5,972,955 and U.S. application serial number 08/772,071 (Atty Docket No. U 011026-1).

Hence, the discovery led to a facile and versatile semi-synthetic methodology by which virtually every camptothecin derivative known in the literature can be transformed into a variety of C-5 substituted camptothecin analogues. Therefore, the present invention provides a novel process for the preparation of various C-5 substituted 20(S)-camptothecin derivatives of the formula where R⁶ has the meaning described above. Also, by virtue of the present invention, a second chiral center at C-5 position was introduced into the camptothecins of the general formula 2 without disturbing the existing 20-hydroxyl group, C-20(S) chiral center. Furthermore, the vast variety of substituents represented by OR⁶ at the C-5 carbon of 20(S)-camptothecins of the formula 1 led to compounds with improved water solubility ranging from 1mg to 10mg per ml. All of the compounds prepared by the present invention exhibited significant in vitro anti-tumor activity against a wide range of human tumor cell lines.

### Detailed Description of the Invention

The present invention particularly provides C-5-O-substituted water soluble analogues of 20(S)-Camptothecin having the formula 1, 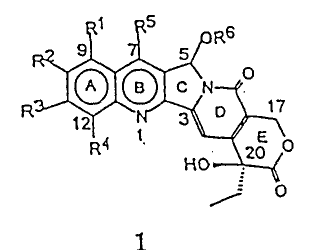 where R¹, R², R³, R⁴, R⁵ and R⁶ have the meaning described above. Throughout the present invention, the terms representing R¹ through R⁶ in these compounds have the following definitions.

The term 'lower alkyl' denotes a univalent, branched or straight hydrocarbon chain containing 1 to 8 carbon atoms. Representative of the alkyl groups are methyl, ethyl, propyl, isopropyl, butyl, sec.butyl tert.butyl pentyl, iso pentyl tert.pentyl, hexyl, isohexyl and octyl.

The term 'lower alkenyl' represents a branched or straight hydrocarbon chain having sp or sp² carbon centers containing 1 to 8 carbon atoms. Representative of the alkenyl groups are vinyl, propenyl, butenyl pentenyl, isopropenyl, isobutenyl, proparginyl, hexenyl and octenyl.

The term 'halogen' or 'halo' represents chlorine, bromine or fluorine. The term 'haloalkyl' denotes alkyl groups substituted with halogens, preferably fluorine, bromine or chlorine. Representative of the haloalkyl groups are chloroethyl, bromopropyl, fluoroethyl, trifluoroethyl, trichloroethyl and trifluorobutyl.

The term 'lower alkoxy' denotes lower alkyl groups as defined above attached via oxygen linkage to the rest of the molecule. Representative of those groups are methoxy, ethoxy; isopropoxy, tert.butoxy, hexoxy, heptoxy and octoxy.

The term 'lower alkanoyl' denotes lower alkyl or alkenyl groups as defined above attached via a carbonyl group to the rest of the molecule. Representative of those groups are acetyl, propionyl, propenoyl, crotanoyl, butanoyl, pentanoyl and isopentanoyl.

The term 'aminoalkyl' represents the lower alkyl groups as defined above substituted with amino groups. Representative of the aminoalkyl groups are 2-aminopropyl, 4-aminobutyl, 5-aminopentyl. Amino groups may also be mono or disubstituted and the representative of these substituted amino groups are dimethylamino, diethylamino, dibenzylamino, ethylisopropylamino, pyrrolidino, piperidino, morphilino or piperizino.

The term 'heteroatom' refers to oxygen, nitrogen or sulfur. The term 'aryl or heteroaryl' represents the groups of aromatic nature having 5 or 6 membered rings which may be selected from phenyl, biphenyl, naphthyl, pyridyl, quinoline, isoquinoline, indole, pyroll, furan, benzofuran, thiophene, pyramidine, piperizine, thiosolidine or imidazole.

The term 'substituted phenyl' group used in the present invention refers to those substituents which can be selected from the groups such as hydroxyl, lower alkyl, haloalkyl, phenyl, benzyl, halogen, lower alkoxy, thioalkoxy, benzyloxy, carboxyl, cyano, nitro, amido, amino, and alkylamino. Examples of such groups are 4-hydroxyphenyl, 3-methoxyphenyl, 4-fluorophenyl, 4-trifluoromethylphenyl, N,N-dimethylaminophenyl, and 4-carbomethoxyphenyl.

The term 'substituted alkyl' group used in the present invention refers to those substituents which can be selected from the groups such as hydroxyl, alkyl, haloalkyl, phenyl, benzyl, halogen, alkoxy, thioalkoxy, benzyloxy, carboxyl, carbonyloxy, cyano, nitro, amido, amino, and alkylamino. Examples of such groups are fluoroethyl, chloropropyl, hydroxyethyl, methoxypropyl, N,N-diethylaminoethyl, N-benzoylaminopropyl, trifluoroethoxyethyl, phenoxyethyl, carbomethoxyethyl, (p-fluorobenzoyloxy)ethyl, aminopropyl, and 2-thioethyl.

The term 'substituted amino' group used in the present invention refers to those substituents which can be selected from the groups such as hydroxyl, alkyl, haloalkyl, benzyl, benzoyl, alkoxy, carboxyl, amido, amino, and alkylamino. Examples of such groups are N,N-diethylamino, N-benzoylamino, N-methoxyamino,

N-carboethoxyamino, and N-chloroethylamino groups. Also, both the substituents on the amino group can be combined together to form 5 or 6-membered cyclic ring system represented by pyrrolidino, piperidino, piperizino, morphilino, imidazolino, or thiazolidino.

According to the present invention there is provided a process for the preparation of the compounds of the general formula 1, 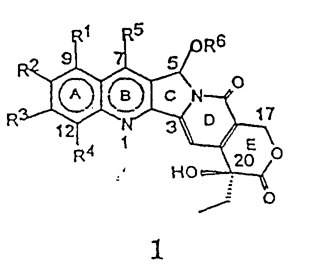 wherein R¹, R², R³, R⁴ are independently the same or different and represent hydrogen, hydroxy, aryloxy, lower alkoxy, lower alkanoyl, nitro, cyano, halo, carboxy, carbonyloxy, amino, substituted amino, lower alkyl, substituted lower alkyl or R², R³ together represent -O-(CH₂)ₙ-O- where n=1 or 2; R⁵ represents hydrogen, lower alkyl, substituted lower alkyl, lower aralkyl, hydroxymethyl, carboxymethyl, aminomethyl, substituted aminomethyl where the amino group may be mono or disubstituted in which both substituents are independent or combined together to form a cyclic ring system of a total of 5-6 atoms containing carbon and optionally one or two heteroatoms selected from oxygen, nitrogen or sulfur; and R⁶ represents hydrogen; phenyl or benzyl where the phenyl group may be unsubstituted or substituted with mono, di or trisubstituents which may be selected from halogen, hydroxy, lower alkoxy, cyano, carboxyl, nitro, amino or substituted amino, lower alkyl, substituted lower alkyl; cycloalkyl or cycloalkyl lower alkyl where the cyclic ring is in the range of 3 membered to 7 membered ring system containing all carbon atoms; lower alkyl groups substituted with heterocyclic rings where the heterocyclic ring system has a total of 3 to 7 atoms, the ring system contains carbon with at least one heteroatom such as oxygen, nitrogen or sulfur; lower alkanoyl; benzoyl where the phenyl group can be unsubstituted or substituted; lower alkenyl; lower alkyl; substituted lower alkyl, substituted lower alkenyl or substituted lower alkanoyl where the substituents can be halogen, hydroxy, lower alkoxy, aryloxy, thio, thioalkyl, thioaryl, aryl or heteroaryl, carboxy, cyano, nitro, amido or amino in which the amino group can be unsubstituted or mono, or disubstituted in which both substituents are independent or combined together to form 5 or 6 membered cyclic ring system containing carbon, and optionally contains one or two heteroatoms selected from oxygen, nitrogen or sulfur, the total number of atoms in the cyclic ring system being 5 or 6; with the proviso that (i) when R¹ is methoxy group, R⁶ is not hydrogen or lower alkyl group; (ii) when R² is hydroxy, lower alkoxy, thioalkyl, nitro, amino, alkylamino, acylamino, and halogen, R⁶ is not hydrogen or lower alkyl group ; (iii) when R⁵ is lower alkyl, lower aralkyl, CH₂OH, COOH, COOMe, or CH₂OR" where R" represents lower alkyl or acyl group, R⁶ is not hydrogen or lower alkyl group (iv) when R¹ is methoxy group, R² is hydroxy, lower alkoxy, thioalkyl, nitro, amino, alkylamino, acylamino, or halogen, R⁵ is lower alkyl, lower aralkyl, CH₂OH, COOH, COOMe or CH₂OR" where R" represents lower alkyl or acyl group, R⁶ is not hydrogen or lower alkyl group; (v) when R¹ through R⁵ represent hydrogen, R⁶ is not hydrogen or lower alkyl group, which comprises,
(i) reacting the compounds of the formula 2, 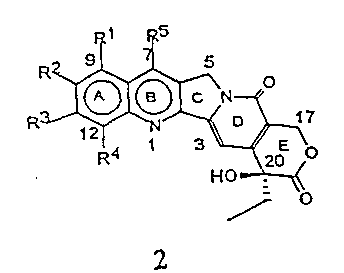 where R¹ to R⁵ have the meaning described above, in the presence of an acid and an oxidizing agent which is a ferric salt, with a compound having the formula R⁶-OH where R⁶ represents lower alkyl, lower alkenyl, (C₃-C₇)cycloalkyl, haloalkyl or hydroxyalkyl, to obtain compounds of the formula 12 and compounds of the formula 13, 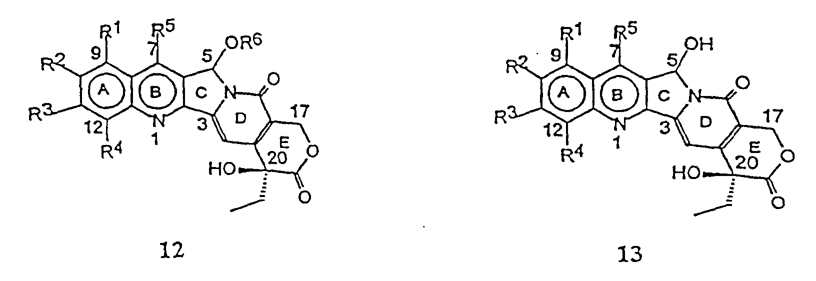 wherein R¹, R², R³, R⁴, R⁵ have the meaning given above,
(ii) separating the compounds of the formulae 12 and 13 prepared in the step (i), by conventional methods,
(iii) hydrolyzing the compounds of the formula 12, by conventional methods, to obtain additional amounts of the compounds of the formula 13,
(iv) reacting the compound of the formula 13, in the presence of an acid, with a compound having the formula R⁶-OH to obtain compounds of the formula 1, 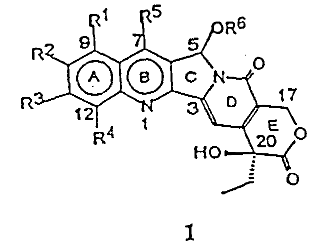 where R¹, R², R³, R⁴ and R⁵ have the meaning described above and R⁶ is as defined above.

According to another feature of the present. invention there is provided an alternate process for the preparation of known C-5 substituted compounds of the formula 1, 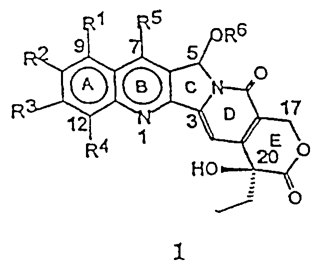 wherein R⁶ represents hydrogen or lower alkyl, R¹ represents hydrogen or methoxy; R² represents hydrogen, hydroxy, lower alkoxy, acyloxy, thioalkyl, SH, thioacyl, nitro, amino, alkylamino, acylamino and halogen; R³ and R⁴ are hydrogen and R⁵ represents hydrogen, lower alkyl, lower aralkyl, CH₂OH, COOH, COOMe or CH₂OR' where R' represents lower alkyl or acyl group which comprises,
(i) reacting the compounds of the formula 2, 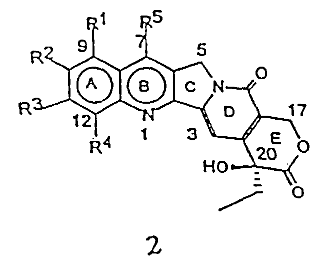 where R¹ to R⁵ have the meaning described above, in the presence of an acid and an oxidizing agent such as ferric salt, with a compound having the formula R⁶-OH where R⁶ represents lower alkyl groups, to obtain compounds of the formula 12 and compounds of the formula 13, 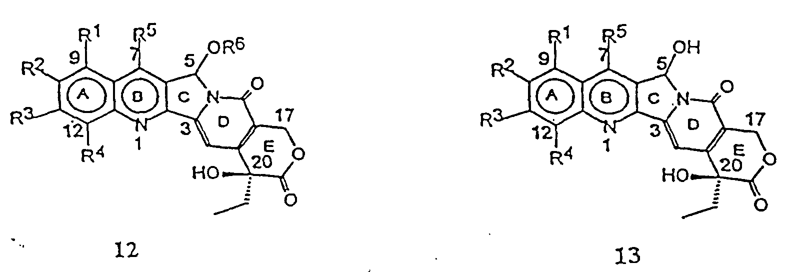 wherein R¹, R², R³, R⁴ and R⁵ have the meaning given above,
(ii) separating the compounds of the formulae 12 and 13 prepared in the step (i), by conventional methods,
(iii) hydrolyzing the compounds of the formula 12, by conventional methods, to obtain additional amounts of the compounds of the formula 13,
(iv) reacting the compound of the formula 13, in the presence of an acid, with a compound having the formula R⁶-OH to obtain compounds of the formula 1, R⁶ represents lower alkyl groups, R¹ represents hydrogen or methoxy, R² represents hydrogen or hydroxy, lower alkoxy, acyloxy, SH, thioalkyl, thioacyl, nitro, amino, alkylamino, acylamino and halogen; R³ and R⁴ are hydrogen and R⁵ represents hydrogen, lower alkyl, lower aralkyl CH₂OH, COOH, COOMe or CH₂OR' where R' represents lower alkyl or acyl group.

The methodology developed and described in the present invention has generated a new chiral center at C-S position in the compounds of formula 2 without disturbing the integrity of 20(S)-∝-hydroxy E-ring lactone moiety. The process developed constitutes a novel, facile and versatile semi-synthetic method for the preparation of C-5 substituted known and novel camptothecin derivatives of the formula 1, starting from the compounds of formula 2. The compounds of the formula 1 prepared by the process of the present invention thus represents diastereomers containing the newly created C-5 chiral center. Indeed, the compounds of the general formula 1 are isolated as a mixture of 20(S),5(R) and 20(S),5(S) diastereomers. However, by the application of conventional analytical techniques, the two diastereomers have also been separated into their single optically pure entities.

In general, all of compounds of the formula 1 where R¹, R², R³, R⁴, R⁵ and R⁶ have the meaning described above, may be synthesized starting from the compounds of the formula 2 by the process described above and can be illustrated with the examples described in the Examples Section. The preparation of the compounds of the formula 12, where R¹, R², R³, R⁴, R⁵ and R⁶ have the meaning given above, from the compounds of the formula 2 as mentioned in the step (i), is a novel transformation in which a direct introduction of various types of alkoxy substituents at C-5 position has been achieved.

The A ring or A/B ring substituted 20(S)-camptothecin derivatives of the general formula 2 where R¹, R², R³, R⁴ and R⁵ have the meaning described above, used as starting materials in the present invention are widely known and prepared according to the prior art documented in the literature. For example, 7-ethylcamptothecin, 10-hydroxycamptothecin, 9-nitrocamptothecin, 12-nitrocamptothecin, 10-hydroxy-7-ethylcamptothecin (SN-38), 9-amino-camptothecin, 9-methoxycamptothecin, 9-hydroxycamptothecin, 9-methoxy-7-ethylcamptothecin, 9-hydroxy-7-ethylcamptothecin, 10,11-methylenedioxycamptothecin, 10,11-ethylenedioxycamptothecin, 10-hydroxy-9- (N,N-dimethylaminomethyl)camptothecin were prepared according to the known literature methods [T. R. Govindachari et al., *Ind. J*. *Chem.* 10(B), 453(1972); S. Sawada et al, *Chem. Pharm. Bull*, 39(10) 2574 (1991), ibid., 39(12), 3183 (1991); US patent no. 4,604,463; and US 4,545,880; Jaffery L. Wood et. al., *J. Org. Chem.*, 60 5739 (1995)] and used as starting materials for the preparation of novel C-ring substituted 20(S)-camptothecin analogues of the general formula 1 described in the present invention.

For example, compounds of the formula 12 where R¹, R², R³, R⁴, R⁵ and R⁶ are independently the same or different and have the meaning given above, can be prepared, as mentioned in the step (i), by the reaction of the compounds of the formula 2 with the compounds having the formula R⁶-OH where R⁶ represents hydrogen, lower alkyl, lower alkenyl, haloalkyl, hydroxyalkyl, (C₃-C₇) cycloalkyl, in the presence of a strong acid and a ferric salt. The acids used in this reaction can be selected from perchloric acid, hydrochloric acid, nitric acid, sulfuric acid or Lewis acids such as boron-trifluoride, zinc chloride, tinchloride, titanium tetrachloride. The ferric salt used in the above reaction can be chosen from ferric nitrate, ferric ammonium sulfate, ferric chloride. In general, the above reaction may be affected at a temperature in the range of 40-150°C., preferably 60 to 120°C.

In step (ii) of the process of the present invention, to separate the mixture of compounds of formulas 12 and 13 as prepared in the step (i) the mixture is subjected to preferably either crystallization or column chromatography technique using silica gel. The solvent mixtures used in the above mentioned methods may contain a combination of the organic solvents such as chloroform, ethyl acetate, methanol, ethanol, ether, acetone and hexane.

The compounds of the formula 13 can also be obtained in step (iii) of the process of the present invention, by treating the compounds of the formula 12 with acids in combination with water at a temperature in the range of 40-120°C. The acids used for this purpose may be selected from hydrochloric acid, hydrobromic acid, sulfuric acid, p-toluenesulfonic acid, acetic acid and perchloric acid. The solvents used in the reaction may be methanol, ethanol, butanol, isopropanol or 1,4-dioxane.

In step (iv) of the process of the present invention, compounds of the formula 13 were reacted with compounds of the formula R⁶-OH where R⁶ has the meaning described above, in the presence of an acid medium at a temperature in the range of 20 to 140°C. to furnish the compounds of the formula 1. The acids used in the reaction may be selected from sulfuric acid, hydrochloric acid, acetic acid, p-toluenesulfonic acid, pyridinium-p-toluenesulfonic acid, camphorsulfonic acid, methanesulfonic acid, perchloric acid or Lewis acids such as titanium tetrachloride, BF₃-etherate and zinc chloride. The solvents used in the reaction may be selected from hexane, benzene, toluene, xylene, chloroform, carbon tetrachloride, dichloroethane, dichloromethane and 1,4-dioxane.

Thus, the present invention is of particular significance in developing C-5-substituted 20(S)-camptothecin derivatives as a new class of C-ring modified camptothecin analogues which are useful as anti-tumor and/or anti-viral agents. The present invention is also of particular significance as the process developed and described here is highly versatile and amenable for large scale preparation of these camptothecin derivatives having the general formula 1.

The methodology developed and described in the present invention will provide access to a wide variety of C-5 substituted C-ring analogues having diverse substituents on rings A and B of 20(S)-campto-thecin. Some of the preferred compounds are those where R¹ is nitro, amino, aminoalkyl, hydroxy, methoxy; R² is hydroxy, carbonyloxy, halo; R², R³ combined together to represent methylenedioxy or ethylenedioxy; R⁴ is hydrogen or nitro; R⁵ is ethyl, aminomethyl or substituted aminomethyl; R⁶ is 2'-hydroxyethyl, alkoxyethyl, chloroethyl, fluoroethyl, trifluoro-ethyl, or aminoethyl or aminopropyl where amino group may be dimethylamino, diethylamino, pyrollidino, piperidino, morphilino, piperizino, imidazolino;
Representative of the compounds of formula 1 are:
1) 5-methoxy CPT*
2) 5-ethoxy CPT*
3) 5-butoxy CPT*
4) 5-chloroethoxy CPT
5) 9-methoxy-5-ethoxy CPT
6) 9-hydroxy-5-ethoxy CPT
7) 10-hydroxy-5-ethoxy CPT*
8) 7-ethyl-5-ethoxy CPT*
9) 7-ethyl-5-hydroxy CPT*
10) 9-nitro-5-ethoxy CPT
11) 9-nitro-5-hydroxy CPT
12) 7-Ethyl-5-chloroethoxy CPT
13) 10-hydroxy-7-ethyl-5-ethoxy CPT
14) 5-(2'-hydroxyethoxy) CPT
15) 7-ethyl-9-hydroxy-5-ethoxy CPT
16) 10-hydroxy-5-(2'-hydroxyethoxy) CPT
17) 7-ethyl-10-hydroxy-5-(2'hydroxyethoxy) CPT
18) 9-nitro-5-fluoroethoxy CPT
19) 9-nitro-5-trifluoroethoxy CPT
20) 10-hydroxy-5-trifluoroethoxy CPT
21) 7-ethyl-10-hydroxy-5-trifluoroethoxy CPT
22) 7-ethyl-5-pyrrolidinoethoxy CPT
23) 7-ethyl-5-dimethylaminopropoxy CPT
24) 7-ethyl-10-hydroxy-5-fluoroethoxy CPT
25) 5-(2'-hydroxyethoxy)-7-ethyl CPT
26) 5-(2'-methoxyethoxy) CPT
where CPT refers to 20(S)-camptothecin
and * represents known compounds in the literature.

Most of the compounds prepared by the present invention have water solubility ranging from 1 mg to 10 mg per ml at 37°C. Table 1A shows MTD in Swiss Albino mice, Lactone Stability in whole blood (after 3 hours), Solubility, Pharmacokinetics MTD, and In vitro activity after 1 hour exposure, for the compounds of Examples 11, 26 and 27.

The protocols used for conducting the experiments are:
1. MTD in Swiss albino mice:
   Each Swiss albino mouse is injected with a single does of the test compound on a day designated as Day 1: Doses that were tested are 400, 200, 100, 50, 25, 12.5, 8.3, 6.25 and 3.13 mg/kg body weight. The animals were observed for mortality and morbidity daily and the body weights of surviving animals were recorded on days 1, 5, 10 and 14. The maximum tolerated dose is defined as the dose at which the test compound did not exhibit any morbidity and body weight reduction more than 30% as compared to day one. (As per the protocol followed by the U.S. National Cancer Institute)
2. Lactone Stability in whole blood:
   2 ml of blood from a healthy volunteer was collected in a tube containing 40 *µ*l of heparin (572 IU) to prevent coagulation, 4 mM and 40 *µ* M working solutions of the drug in DMSO were prepared and added to aliquots of whole blood to give final concentration of 100 *µ* M and 1 *µ*M respectively. The drug is incubated in whole blood at 37°C. and 20 *µ*l samples are collected into 180 *µ*l of chilled methanol (-30°C.) at different time intervals (0, 1, 2 & 3 hrs). Vortex and then centrifuge at 11000 rpm for 3 min in a microcentrifuge at room temperature. Dilute 100 *µ*l of the supernatant with water to 300-500 *µ*l depending upon the signal response (UV for 100 *µ*M concentration and fluoroscence for 1 *µ*M concentration) of the compound. 200 *µ*l of the diluted sample is injected on to the HPLC column previously equilibrated with the mobile phase. The area under peak corrsponding to lactone forms is measured. Zero time peak area is taken as 100%, and the proportion of lactone peak area at different time points is calculated to determine the equilibrium lactone stability as obtained by consistent lactone proportion over two successive time points. (*Biochemistry*, 1994; 33:10325-10336 and *J. Pharm. Sci.*, 1995; 84:518-519).
3. Pharmacokinetics at MTD:
   All studies were carried out in Swiss albino mice in the weight range 35-40 g. The animals were fasted overnight prior to dosage of the drug and were fed 3 hours after dosing. The animals were dosed intra-peritonially as a solution in DMSO:water (50:50; v/v). Blood samples were drawn from orbital sinus at 1, 2, 4, 6 and 8 hours after administration of the dose into heparinized tubes, centrifuged at 13000 RPM for 10 min. Plasma samples were separated and analyzed by HPLC. To 50 *µ*l of the sample, 100 *µ*l of chilled acidified methanol was added and mixed to precipitate proteins. The sample was centrifuged at 13,000 RPM for 10 min. 100 *µ*l of supernatant was made up to 200 *µ*l with methanol:water (50:50; v/v) and 100 *µ*l was injected on HPLC. Peak area of the drug was used for quantification. Calibration, control and recovery samples were prepared by spiking 50 *µ*l of blank plasma with known amounts of the drug and processed in the same manner as the samples. (*J. Natl. Cancer Inst.,* 1996; 88:817-824).
4. Solubility by HPLC method:
   Excess of compound was soaked in 0.5 ml of 0.1M sodium acetate buffer at pH 5.0 for 24 hours at room temperature. The solution was filtered through 0.45 micron PVDF syringe filter (Gelman Sciences). The filtrate was injected into HPLC at different volumes (10 & 20 *µ*l). Chromatograms were recorded. Responses recorded were extrapolated from the calibration curve and the solubility of the compound was calculated. (*J*. *Med. Chem*., 1995; 38: 400)
5. Solubility by routine method:
   Compound was suspended in 5 ml of deionized water and heated to 37°C. for 10 min. Then, the solution was filtered and the filtrate was evaporated to drying using methanol and the solid residue was weighed.
6. In vitro activity after 1 hour exposure:
   Grow the cells in 15 ml of Complete Medium (RPM1-1640 with 10% Fetal bovine serum and 0.2% NaHCO₃) for 3-5 days to obtain a cell number of 10⁶ cells/flask. The medium is removed and the attached cells are washed with Phosphate Buffered Saline (PBS). 1 ml of 0.1% Trypsin-EDTA is added and incubated for 5 min at 37°C. Tap the flasks gently and add 5 ml of complete medium. Remove cell suspension and centrifuge at-2000 rpm for 5 min. Discard the supernatant and suspend the pellet in 5 ml of complete medium. Count the cell number in a haemocytometer. Dilute the cell suspension to 10,000 cells/100 *µ*l in complete medium. Plate out 100 *µ*l of cell suspension in each of 96 well microtitre plate and incubate for 24 hrs at 37°C. and 5% CO₂. Terminate the reference blank (plated out separately) with 25 *µ*l of 50% cold Trichloroacetic acid (TCA). Incubate for 1 hour at 4°C. Wash the plate (five times) with deionized water. Air dry and preserve the plate at 4°C. for determination of T₀ value. Prepare suitable dilution of the test compound in complete medium and add 100 *µ*l to each well to maintain the final concentration ranging between 10⁻⁴M and 10⁻⁸M. Incubate for 1 hr at 37°C. and 5% CO₂. Centrifuge the microtitre plate at 1000 rpm for 5 min. Remove the supernatant. Wash the cells twice with 100 *µ*l of PBS to remove the traces of test compound. Add 200 *µ*l of complete medium to each well and incubate for 48 hours at 37°C. and 5% CO₂. Terminate the cell growth with the addition of 50 *µ*l of 50% cold TCA. Incubate the plate for 1 hr at 4°C. Wash the plates with deionized water (five times) and air dry. Add 100 *µ*l of Sulforhodamine B solution (0.4% in 1% acetic acid) to each well. Keep at room temperature for 15 minutes. Wash (five times) with 1% acetic acid and air dry. Add 100 *µ*l of 10mM Trizma base (Sigma), shake gently on plate shaker for 15 minutes and read the optical density at 490 nm in Spectrophotometric plate reader. (as per the protocol followed by the U.S. National Cancer Institute.

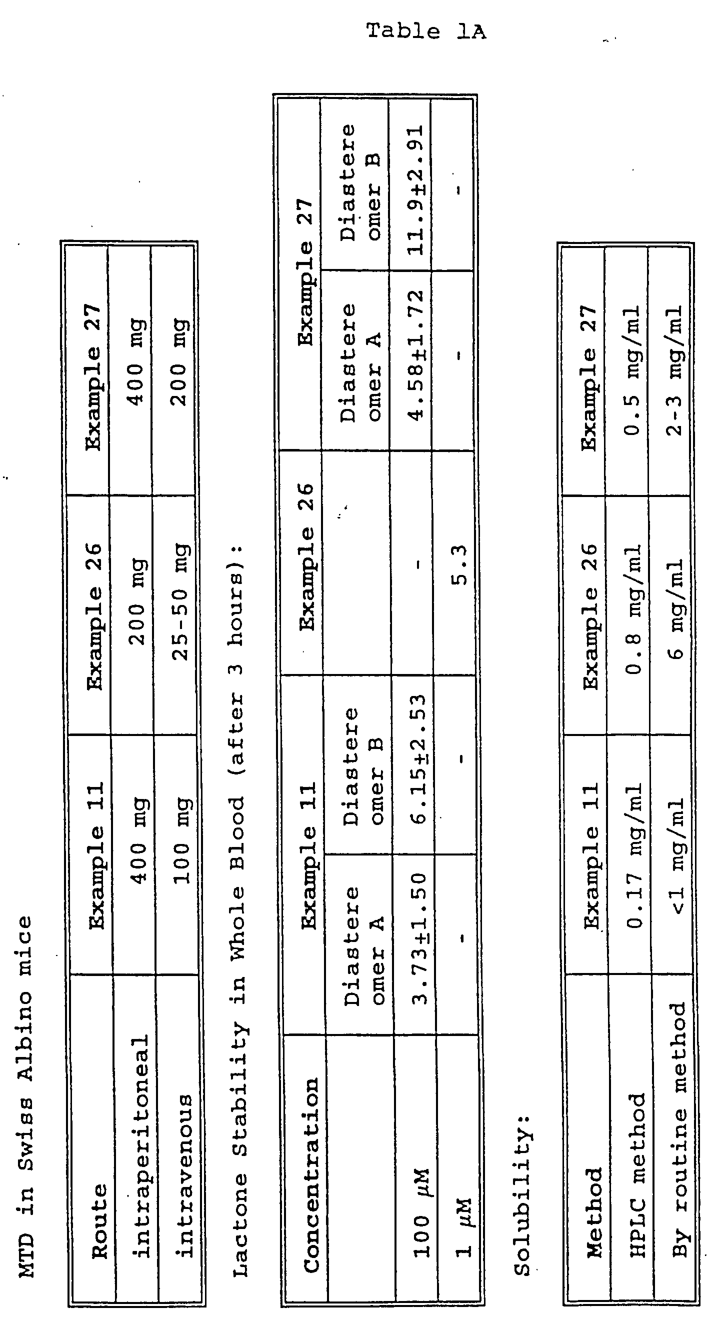

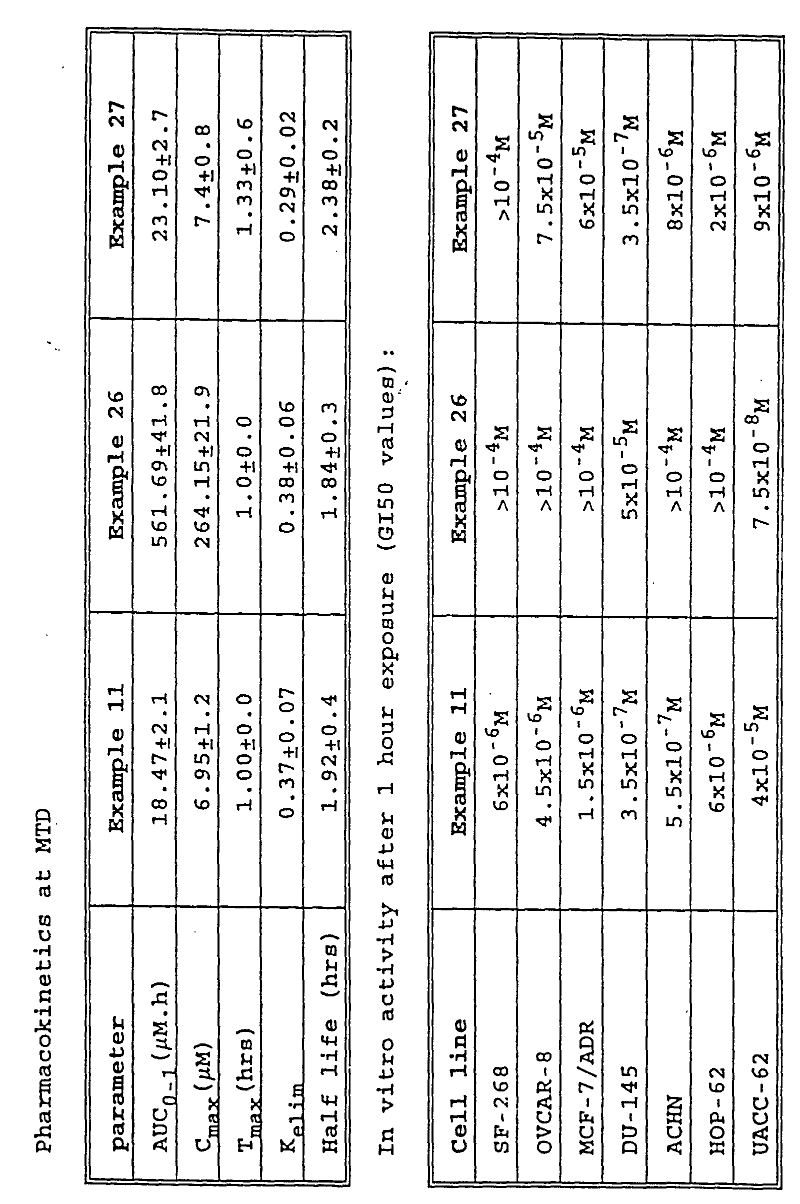

Further, several compounds prepared in the present invention exhibited good in vitro anti-cancer activity towards various human tumor cell lines, according to the results obtained from the 60 human tumor cell line assay performed at National Cancer Institute (NCI), Bethesda, Maryland, U.S.A

Table 1 presents in vitro cell line activity expressed as IC50 values for various 20(S)-camptothecin C-ring analogues prepared in the present invention. Charts 1 to 3 present the data compiled based on NCI mean graphs for total growth inhibition (TGI) of different types of human cancer cell lines for the compounds prepared in the examples 27, 28 and 43. Similar data compiled for topotecan based on NCI mean graph is also included for comparison purposes. The data presented in Tables 2 and 3 shows that the C-ring analogues of 20(S)-camptothecin prepared in the present invention exhibited anti-tumor activity equal or superior to topotecan towards certain cell lines of different cancer cell panels. Table 4 presents the data obtained for the compound prepared in the example 32 against AIDS related lymphoma(ARL) cell lines. All the compounds used in the NCI's in vitro anti-cancer screening programme are mixtures substantially containing both the diastereomers having 20(S),5(S) and 20(S),5(R) configurations in varied ratios.

The results shown in charts 1 to 3 and tables 1 to 4 were obtained from conducting experiments according to U.S. National Cancer Institute (NCI) protocols as given below:

Each test compound was screened against a battery of 60 human cell lines obtained from eight organs. In a typical procedure, the cell suspensions that were diluted according to the particular cell type and the expected target cell density (5000-40,000 cells per well based on cell growth characteristics) were added into 96-well microtiter plates. Inoculates were allowed a preincubation period of 24h at 37°C. for stabilization. Dilutions at twice the intended test concentrations were added at time zero in 100-µl aliquots to microtiter plate wells. Usually test compounds were evaluated at five 10-fold dilutions. The highest well concentration used in the test is 10⁻⁴M. The cells are then incubated in the presence of drug (the test compound) for further 48h in 5% CO₂ atmosphere and 100% humidity. At the end of this time, the adherent cells are fixed to the plate by means of trichloroacetic acid, and after a number of washes, the cell layer is treated with the protein stain Sulforhodamine B. The optical density which is proportional to protein mass, is then read by automated spectrophotometric plate readers at a wavelength of 515 nm. Readings are transferred to a microcomputer and final reports are generated using especially developed software.

The compounds of formula 1 of the present invention, and the pharmaceutically acceptable salts thereof as described above, and the compositions containing them, are useful as anti-cancer and anti-viral agents. Administration of the novel active compounds of the formula 1, in pure form or in an appropriate pharmaceutical composition can be carried out via any of the accepted modes of administration for serving similar utilities. Thus, administration can be, for example, orally, nasally, parenterally or topically, in the form of solid, semi-solid, lyophilized powder, or liquid dosage forms, such as for example, tablets, suppositories, pills, capsules, powders, solutions, suspensions, emulsions, creams, lotions, aerosols, ointments, injections or the like, preferably, in unit dosage forms suitable, for simple administration of precise dosages. The compositions will include a conventional pharmaceutical carrier, diluent or excipient and an active novel compound of formula 1 and, in addition, may include either medicinal agents, pharmaceutical agents, carriers, adjuvants, etc.

The invention is described in detail with specific examples given below, which are provided by way of illustration only and should not be considered to limit the scope of the invention.

**TABLE 1:**

| S. NO. | COMPOUND | IC50 (µm)^{a} |
|---|---|---|
| 1. | 5-Methoxycamptothecin* | 8.5 |
| 2. | 5-Ethoxycamptothecin* | 9.54 |
| 3. | 5-n-Butoxycamptothecin* | 6.16 |
| 4. | 5-(2'-Hydroxyethoxy)camptothecin | 1.51 |
| 5. | 5-(2'-Chloroethoxy)camptothecin | 4.57 |
| 6. | 7-Ethyl-5-ethoxycamptothecin* | 1.41 |
| 7. | 9-Methoxy-7-ethyl-5-ethoxycamptothecin | 2.13 |
| 8. | 7-Ethyl-5-chloroethoxy camptothecin | 2.75 |
| 9. | 7-Ethyl-5-aminoethoxy camptothecin | 18.6 |
| 10. | 7-Ethyl-5-pyrollidinoethoxy camptothecin | 18.6 |
| 11. | 7-Ethyl-5-piperidinoethoxy camptothecin | >30 |
| 12. | 7-Ethyl-5-N,N-dimethylaminoethoxy | |
| | camptothecin | 13.8 |
| 13. | 7-Ethyl-5-N,N-dimethylaminopropoxy | |
| | camptothecin | >30 |
| 14. | 9-Methoxy-5-ethoxy camptothecin | 2.45 |
| 15. | 5-Trifluoroethoxy camptothecin | 1.82 |
| 16. | 5-Aminoethoxy camptothecin | 30.0 |
| 17. | 7-Ethyl-5-trifluoroethoxycamptothecin | 7.41 |
| 18. | 7-Ethyl-5-(2'-hydroxyethoxy)camptothecin | 4.78 |
| 19. | 5-Fluoroethoxycamptothecin | 1.58 |
| 20. | 10-Hydroxy-5-trifluoroethoxy camptothecin | 0.38 |
| 21. | 9-Nitro-5-trifluoroethoxy camptothecin | 0.46 |
| 22. | 10-Hydroxy-5-(2'hydroxyethoxy)camptothecin | 8.12 |
| 23. | 9-Nitro-5-(2'-hydroxyethoxy)camptothecin | 7.94 |
| 24. | 7-Ethyl-5-fluoroethoxy camptothecin | 4.36 |
| 25. | 5-Methoxyethoxy camptothecin | 2.23 |
| 26. | 9-Nitro-5-methoxyethoxy camptothecin | 2.04 |
| 27. | 12-Nitro-5-ethoxy camptothecin | >30 |
| 28. | 12-Nitro-5-hydroxy camptothecin | >30 |
| 29. | 9-Amino-5-methoxy camptothecin | 6.76 |
| 30. | 9-Hydrory-5-ethoxy camptothecin | 6.68 |

| | | |
|---|---|---|
| a IC50 = the mean value of the minimum drug concentration (µm) of the agent required to produce 50% cell growth inhibition (GI50) against NCI's 60 human tumor cell line assay. | | |
| * represents C-5 substituted camptothecin derivatives known in the literature. | | |

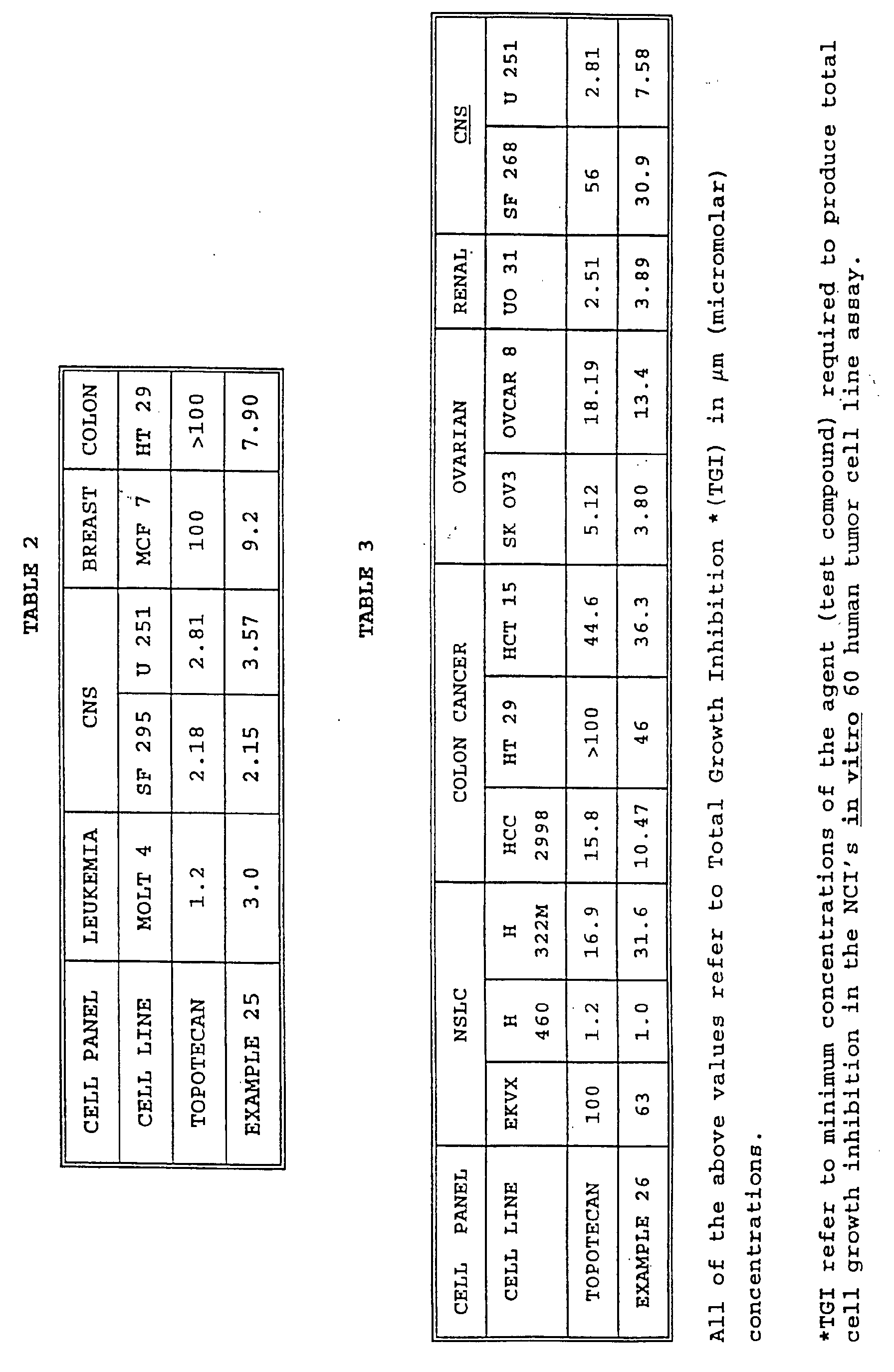

**Table 4#:**

| **IN Vitro activity of Example 32 against AIDS related Lymphoma (ARL) cell lines:** | | |
|---|---|---|
| CELL NAME | GI50* | TGI** |
| CCRF - CEM | 0.318 | 1.83 |
| RL | 0.463 | 3.94 |
| KD 488 | 0.246 | 2.28 |
| AS 283 | 0.268 | 0.678 |
| PA 682 | 0.456 | 7.23 |
| SU-DHL-7 | 0.609 | 3.51 |

| | | |
|---|---|---|
| * GI50 refers to the minimum concentration (µm) of the text compound required for 50% cell Growth Inhibition. | | |
| **TGI refer to minimum concentration (µm) of the text compound required for the Total Growth Inhibition. | | |

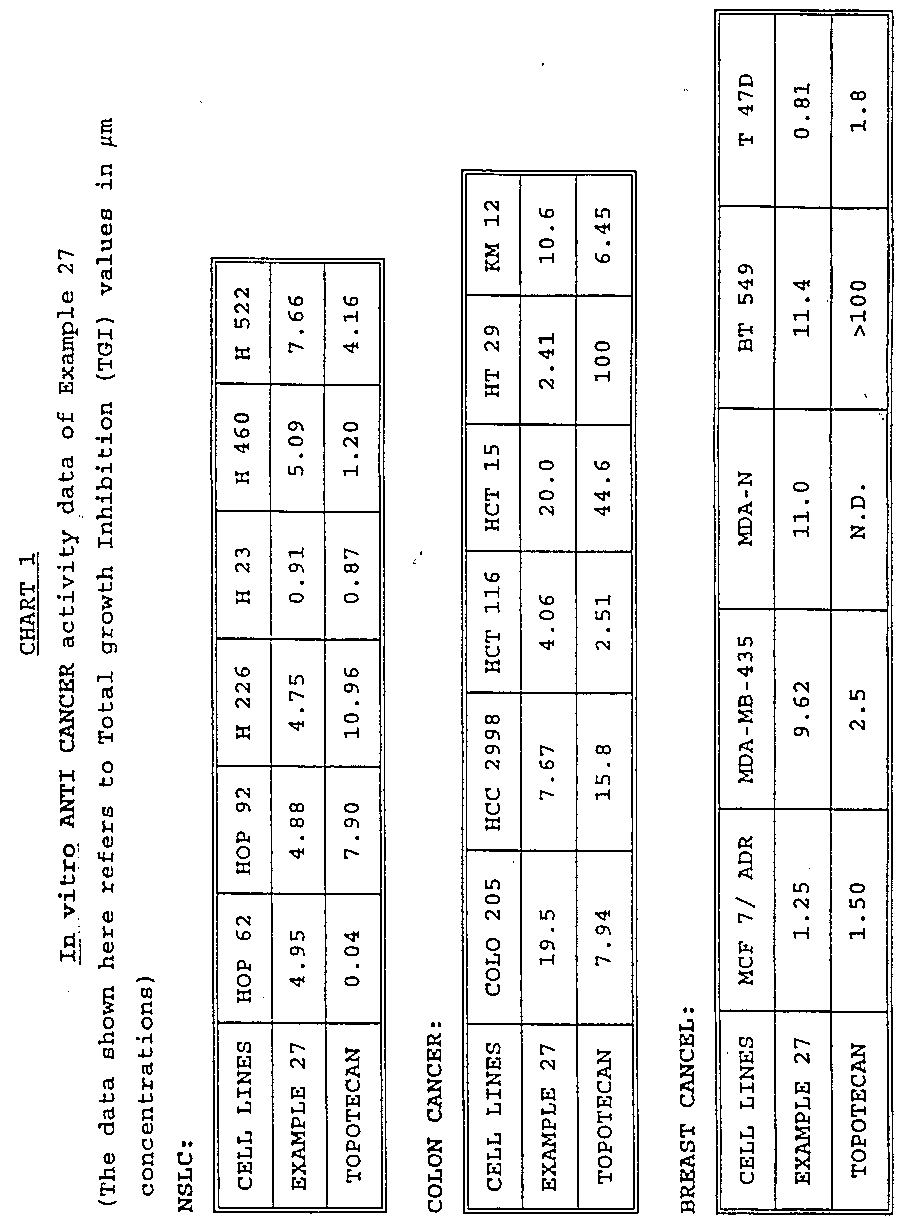

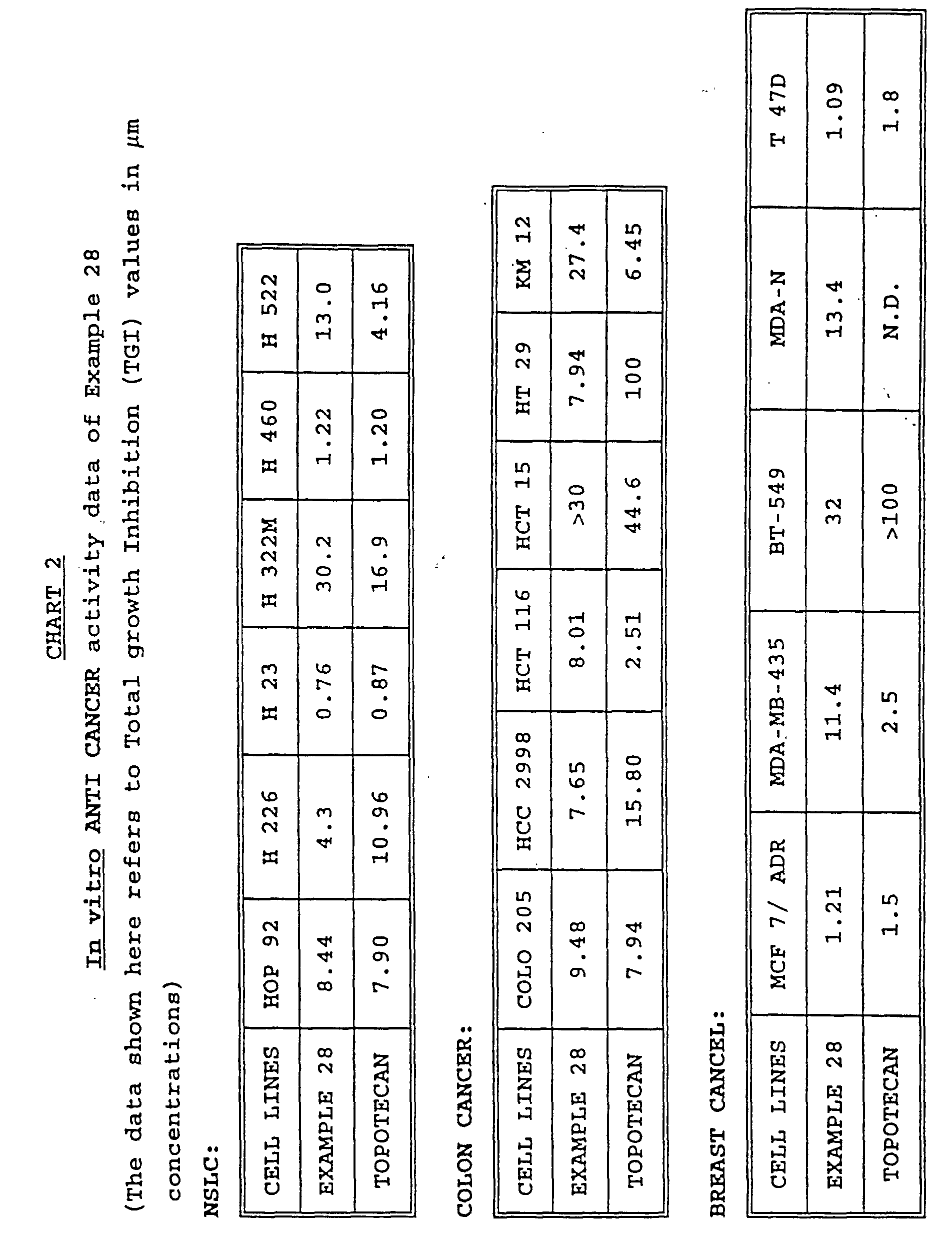

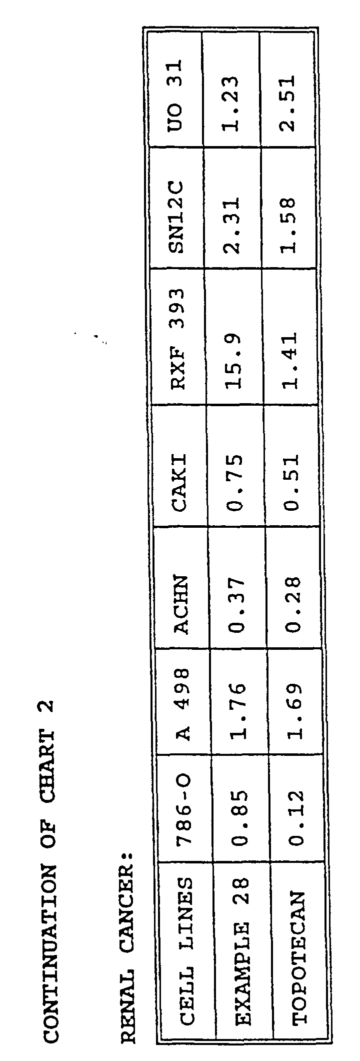

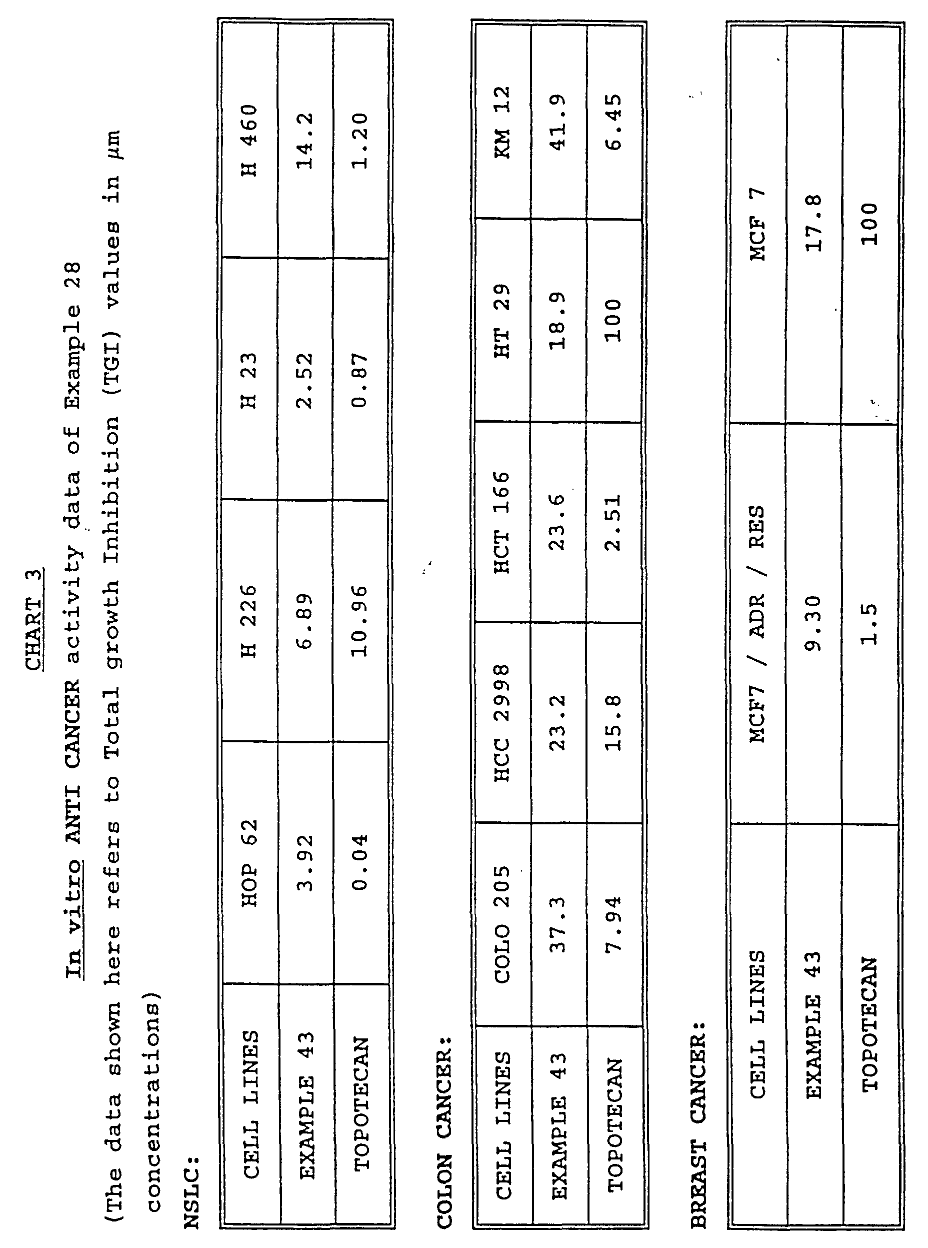

### EXAMPLES

### EXAMPLE 1

### Preparation of 5-methoxycamptothecin (known compound)

**Step 1:** To a mixture of 20(S)-Camptothecin of the formula 3 (2g), ferric chloride (2g), dissolved in 80ml of methanol, 10ml of sulfuric acid was added dropwise and continued heating at 70°C. for 24h. Excess acid and methanol were removed under vacuum and the residue was extracted with ethylacetate. Organic layer was washed with water, brine and dried over anh.sodium sulfate. Concentration of the solvent afforded 1.8g of yellowish powder containing 5-methoxycamptothecin and 5-hydroxy camptothecin in the ratio of 5:1.

**Step 2:** Separation of the mixture by silica gel column chromatography using methanolchloroform solvent mixture as eluent afforded 1.5g of 5-methoxycamptothecin and 300mg of 5-hydroxycamptothecin. Analytical data for 5-methoxycamptothecin: mp: 156°C.; [∝]_{D} at 28°C. = + 41.74 (c 0.103, CHCl₃); IR: 3426, 1747, 1664, 1616, 1228, 1155, 1046, 762 cm⁻¹; ¹H NMR (CDCl₃, 200MHz): δ 8.42(s, 1H), 8.26(d, J=8Hz, 1H), 7.96(d, J=8Hz, 1H), 7.88 (t, J= 6.8Hz, 1H), 7.68(t, J=6.8Hz, 1H), 7.58(s, 0.5H), 7.54(s, 0.5H), 6.95(s, 0.5H), 6.80(s, 0.5H), 5.74(d, J=16.5Hz, 0.5H), 5.72 (d, J=16.5Hz, 0.5H), 5.25(d, J=16.5Hz, 1H), 3.75(s, 1H), 3.70(s, 1.5H), 3.50(s, 1.5H), 2.01-1.82(m, 2H), 1.06(t, J=7Hz, 3H); Mass (m/z): 379(M+H), 348, 319.

### EXAMPLE 2

### Preparation of 5-Hydroxycamptothecin (known compound)

**Step 1:** Preparation of 5-methoxy camptothecin: 5-Methoxycamptothecin of the formula 1 where R¹=R²=R³=R⁴=R⁵=H, R⁶=Me was prepared from 20(S)-camptothecin of the formula 3 as described in the example 1.

**Step 2:** 1.5g of 5-methoxycamptothecin of the formula 1 where R¹=R²=R³=R⁴=R⁵=H, R⁶=Me was dissolved in 50ml of methanol and treated with 50 ml of 50% HCl. The solution was heated to reflux for 30h. At the end, excess water and methanol were removed as an azeotropic mixture and the residue was extracted with ethylacetate. Organic layer was washed with brine and dried over anh.sodium sulfate. Concentration of the solvent afforded 1.2g of 5-hydroxycamptothecin after purification over silica gel column chromatography using a solvent mixture of ethyl acetate and chloform; mp: 220°C.; [∝]_{D} at 26°C. = +28.00 (c 0.1 in CHCl₃); IR: 3367, 1749, 1658, 1591, 1159, 1046 cm⁻¹; ¹H NMR (CDCl₃+DMSO-d6, 200MHz): δ 8.50 (s, 1H), 8.20 (d, J= 8Hz, 1H), 7.94(d, J=8Hz, 1H), 7.85(t, J=6.8Hz, 1H), 7.64(t, J=6.8Hz, 1H), 7.58 (s, 0.5H), 7.56(s, 0.5H), 7.06(s, 0.5H), 7.01(s, 0.5H), 6.95(br d, 1H, D₂O exchangeable), 5.67(d, J= 16.5Hz, 1H), 5.25(d, J=16.5Hz, 1H), 5.05 (br d, 1H, D₂O) exchangeable), 2.05-1.86(m,2H), 1.06(t, J=7Hz, 3H); Mass (m/z): 364(M+1), 348, 320, 277, 236, 91, 57.

### EXAMPLE 3

### Preparation of 5-Ethoxy-7-ethylcamptothecin (known compound)

**Step 1:** To a mixture of 7-ethylcamptothecin of the formula 2 where R¹=R²=R³=R⁴=H, R⁵=Et(1.5g), ferric chloride (1.35g), dissolved in 150ml of ethanol, 9ml of sulfuric acid was added dropwise and continued heating at 85°C. for 30h. Excess acid and ethanol were removed under vacuum and the residue was extracted with ethylacetate. Organic layer was washed with water, brine and dried over anh.sodium sulfate. Concentration of the solvent afforded 1.6g of brownish powder containing 5-ethoxy-7-ethylcamptothecin and 5-hydroxy-7-ethylcamptothecin in the ratio of 10:1.

**Step 2:** Separation of the mixture by column chromatography gave 1g of 5-ethoxy-7-ethyl-camptothecin and 100mg of 5-hydroxy-7-ethylcamptothecin; mp: 150°C; [∝]_{D} at 27°C. = + 10.526 (c 0.085, CHCl₃); IR: 3419, 1751, 1662, 1613, 1157, 1075, 1050, 764 cm⁻¹; ¹H NMR (CDCl₃, 200MHz): δ 8.20(d, J=8Hz, 1H), 8.15(d, J=8Hz, 1H), 7.81(t, 3=6.8Hz, 1H), 7.66(t, 7.3Hz, 1H), 7.54(s, 0.5H), 7.51(s, 0.5H), 7.01(s, 0.5H), 6.89(s, 0.5H), 5.72(d, J=16.5Hz, 0.5H), 5.71(d, J=16.5Hz, 0.5H), 5.28(d, J=16.5Hz, 0.5H), 5.26(d, J=16.5Hz, 0.5H), 4.3-3.6(m, 3H), 3.5-3.1 (m, 2H), 2.05-1.71(m, 2H), 1.45(t, J=7.5Hz, 3H), 1.06(t, J=7Hz, 3H).

### EXAMPLE 4

### Preparation of 5-Hydroxy-7-ethylcamptothecin (known compound)

**Step 1:** Preparation of 5-Ethoxy-7-ethylcamptothecin: 5-Ethoxy-7-ethylcamptothecin of the formula 1 where R¹=R²=R³=R⁴=H, R⁵=R⁶=Et, was prepared from 20 (S)-camptothecin of the formula 2 as described in the example 3.

**Step 2:** 50 ml of 25% H₂SO₄ was added to 1.0g of 5-ethoxy-7-ethylcamptothecin of the formula 1 where R¹=R²=R³=R⁴=H, R⁵=R⁶=Et, dissolved in 30ml of ethanol and heated to reflux for 30h. At the end, excess water and ethanol were removed as an azeotropic mixture and the residue was extracted with ethylacetate. Organic layer was washed with brine and dried over anh.sodium sulfate. Concentration of the solvent afforded 700mg of 5-hydroxy-7-ethylcamptothecin after purification over silica gel column chromatography; mp: 252°C.; IR: 3349, 1752, 1656, 1605, 1159, 1054, 766 cm⁻¹; Partial data of ¹H NMR (CDCl₃ + DMSO-d6) δ 7.19(br s, 1H, D₂O exchangeable), 7.15(s, 0.5H), 7.05(s, 0.5H), 5.75(br s, 1H, D₂O exchangeable), 5.65(d, J=16.5Hz, 1H), 5.25 (d, J=16.6Hz, 1H), 3.52-3.19(m, 2H), 1.45(t, J= 7.5Hz, 3H), 1.02 (m, 3H).

### EXAMPLE 5

### Preparation of 5-Ethoxy-9-methoxycamptothecin of the formula 1 where R¹=OMe, R²=R³=R⁴=R⁵=H, R⁶=Et

**Step 1:** To a mixture of 9-methoxycamptothecin of the formula 2 where R¹=OMe, R²=R³=R⁴=R⁵=H(1g), ferric chloride (500mg), dissolved in 50ml of ethanol 10ml of sulfuric acid was added dropwise and continued heating at 85°C. for 22h. Excess acid and ethanol were removed under vacuum and the residue was extracted with ethylacetate. Organic layer was washed with water, brine and dried over anh.sodium sulfate. Concentration of the solvent afforded dark brownish powder.

**Step 2:** Purification of the above residue over column chromatography using silica gel furnished 500mg of 5-ethoxy-9-methoxycamptothecin of the formula 1 and 300mg of 5-hydroxy-9-methoxycamptothecin; mp: 235°C.; [∝]_{D} at 30°C.= + 34.18 (c 0.093, MeOH); IR: 3436, 748, 1665, 1619, 1461, 1366, 1093, 814cm⁻¹; ¹H NMR (CDCl₃, 200MHz): δ 8.81(s, 1H), 7.78(m,2H), 7.53(d, J=5.5Hz, 1H), 6.96(d, J=7Hz, 1H), 6.88 (s, 1H), 6.77(s, 1H), 5.72(d, J=16Hz, 0.5H), 5.75(d, J=16Hz, 0.5H), 5.27(d, J=16Hz, 1H), 4.24-3.90(m, 2H), 4.06(s, 3H), 3.80(s, 1H, D₂O exchangeable), 1.90(m, 2H), 1.31(m,3H), 1.01(m, 3H); Mass (m/z): 422(M+1), 394, 378, 350, 305, 98, 57.

### EXAMPLE 6

### Preparation of 5-Hydroxy-9-methoxycamptothecin (known compound)

**Step 1:** Initially 5-Ethoxy-9-methoxycamptothecin of the formula 1 where R¹=OMe, R²=R³=R⁴=R⁵=H, R⁶=Et, was prepared as described in the example 5.

**Step 2:** 25 ml of 80% HCl was added to 560mg of 5-ethoxy-9-methoxycamptothecin of the formula 1 where R¹=OMe, R²=R³=R⁴=R⁵=H, R⁶=Et, dissolved in 25ml of ethanol and heated to reflux for 16h. At the end, excess water and ethanol were removed as an azeotropic mixture and the residue was extracted with ethylacetate. Organic layer was washed with brine and dried over anh.sodium sulfate. Concentration of the solvent afforded 520mg of 5-hydroxy-9-methoxy-camptothecin after purification over silica gel column chromatography; mp: 162°C.; [∝]_{D} at 30°C. = + 39.68 (c 0.012, MeOH); IR: 3398, 1749, 1656, 1616, 1577, 1465, 1383, 1154, cm⁻¹; ¹H NMR (CDCl₃+ DMSO-d6): δ 8.81(s, 1H), 7.81-7.61(m, 2H), 7.50(d, J=5.5Hz, 1H), 7.12-6.71 (m, 2H), 5.70(d, J=16Hz, 1H), 5.30(d, J=16Hz, 1H), 4.06(s, 3H), 1.98-1.75(m,2H), 1.10-0.98(m, 3H); Mass (m/z): 394(M+1), 377, 348, 266, 149, 88, 57.

### EXAMPLE 7

### Preparation of 5-Ethoxy-9-methoxy-7-ethylcamptothecin of the formula 1 where R¹=OMe, R²=R³=R⁴=H, R⁵=R⁶=Et

**Step 1:** To a mixture of 9-methoxy-7-ethylcamptothecin of the formula 2 where R¹=OMe, R²=R³=R⁴=H, R⁵=Et(100mg) , ferric chloride (100mg), dissolved in 32ml of ethanol 2ml of sulfuric acid was added dropwise and continued heating at 85°C. for 4h. Excess acid and ethanol were removed under vacuum and the residue was extracted with ethylacetate. Organic layer was washed with water, brine and dried over anh.sodium sulfate. Concentration of the solvent afforded 120mg of residue containing 5-ethoxy-9-methoxy-7-ethylcamptothecin and 5-hydroxy-9-methoxy-7-ethylcamptothecin in the ratio of 1:4.

**Step 2:** Separation of the mixture by column chromatography using the solvent mixture of ethyl acetate-chloroform gave 15mg of 5-ethoxy-9-methoxy-7-ethylcamptothecin of the formula 1 and 55mg of 5-hydroxy-9-methoxy-7-ethylcamptothecin; mp: 240°C.; IR: 3443, 1747, 1663, 1609, 1458, 1254, 1160, 1074 cm⁻¹; ¹H NMR (CDCl₃, 200MHz): δ 7.80-7.68(m, 2H), 7.50(s, 0.5H), 7.47(s, 0.5H), 7.01(s, 0.5H), 6.98(d, J=8Hz, 1H), 6.89(s, 0.5H), 5.76(d, J=16Hz, 1H), 5.28(d, J=16Hz, 0.5H), 5.26(d, J=16Hz, 0.5H), 4.25-3.81(m, 2H), 4.02(s, 3H), 3.72-3.28(m, 2H), 3.15(br s, 1H, D₂O exchangeable), 2.02-1.82(m, 2H), 1.37-1.33(m, 3H), 1.05-0.95(m, 3H); Mass (m/z): 451(M+1), 406, 377, 362, 347, 331, 261, 181, 149, 97.

### EXAMPLE 8

### Preparation of 5-Hydroxy-9-methoxy-7-ethylcamptothecin of the formula 13 where R¹=OMe, R²=R³=R⁴=H, R⁵=R⁶=Et

**Step 1:** Initially 5-Ethoxy-9-methoxy-7-ethylcamptothecin of the formula 1 where R¹=OMe, R²=R³=R⁴=H, R⁵=R⁶=Et, was prepared as described in the example 7.

**Step 2:** 5 ml of 50% HCl was added to 100mg of 5-ethoxy-9-methoxy-7-ethylcamptothecin of the formula 1 where R¹=OMe, R²=R³=R⁴=H, R⁵=R⁶=Et, dissolved in 5ml of ethanol and heated to reflux for 26h. At the end, excess water and ethanol were removed as an azeotropic mixture and the residue was extracted with ethylacetate. Organic layer was washed with brine and dried over anh.sodium sulfate. Concentration of the solvent afforded 80mg of 5-hydroxy-9-methoxy-7-ethylcamptothecin of the formula 13 after purification over silica gel column chromatography; mp: 242°C.; IR: 3440, 1742, 1660, 1610, 1456, 1250, 1160 cm⁻¹.

### EXAMPLE 9

### Preparation of 5-Ethoxycamptothecin (known compound)

**Step 1:** To a mixture of 20(S)-Camptothecin of the formula 3 (1g), ferric chloride (1g), dissolved in 50ml of ethanol 12ml of BF₃-etherate was added dropwise and continued heating at 85°C. for 40h. Excess acid and ethanol were removed under vacuum and the residue was extracted with ethylacetate. Organic layer was washed with water, brine and dried over anh.sodium sulfate. Concentration of the solvent afforded 1g of yellowish powder containing 17-ethoxycamptothecin and 5-hydroxycamptothecin in the ratio of 6:1.

**Step 2:** Separation of this mixture by silica gel column chromatography using ethyl acetate hexane solvent mixture as eluent afforded 700mg of 5-ethoxycamptothecin and 120mg of previously prepared 5-hydroxycamptothecin; mp: 140°C.; [∝]_{D}, at 28°C. = + 29.703 (c 0.101, CHCl₃); IR: 3423, 1746, 1663, 1616, 1155, 1070, 1040 cm⁻¹; Partial ¹H NMR data in CDCl₃: δ 6.9(s, 0.5H), 6.78(s, 0.5H), 4.25-3.85(m, 2H), 3.70(s, 1H), 2.00-1.80(m, 2H), 1.40-1.22(m, 3H), 1.12-0.98(m, 3H); Mass (m/z): 393 (M+1), 378, 362, 348, 319, 247, 219, 57.

### EXAMPLE 10

### Preparation of 5-butoxycamptothecin (known compound)

**Step 1:** To a mixture of 20(S)-Camptothecin of the formula 3(500mg), ferric chloride (500mg), dissolved in 15ml of n-butanol, sulfuric acid was added dropwise and continued heating at 100°C. for 20h. Excess acid and n-butanol were removed under vacuum and the residue was extracted with ethylacetate. Organic layer was washed with water, brine and dried over anh.sodium sulfate. Concentration of the solvent afforded powdery material.

**Step 2:** Purification of the above material by silica gel column chromatography using ethylacetate-hexane solvent mixture as eluent afforded 300mg of 5-butoxycamptothecin and 50mg of previously prepared 5-hydroxycamptothecin; mp: 82°C.; [∝]_{D} at 28°C. = +28.00 (c 0.1, CHCl₃); Partial ¹H NMR data in CDCl₃: δ 6.92(s, 0.5H), 6.79(s, 0.5H), 4.12-3.75(m, 2H), 3.80(br s, 1H, D₂O exchangeable), 2.00-1.82(m, 2H), 1.75-1.52 (m, 2H), 1.50-1.29(m, 2H), 1.15-0.82(m, 6H); Mass (m/z): 422(M+1), 363, 348, 319, 84, 51.

### EXAMPLE 11

### Preparation of 5-Ethoxy-9-hydroxycamptothecin of the formula 1 where R¹=OH, R²=R³=R⁴=R⁵=H, R⁶=Et

**Step 1:** To a mixture of 9-hydroxycamptothecin of the formula 2 where R¹=OH, R²=R³=R⁴=R⁵=H, (200mg), ferric chloride (250mg), dissolved in 40ml of ethanol 1.5ml of sulfuric acid was added dropwise and continued heating at 85°C for 26h. Excess acid and ethanol were removed under vacuum and the residue was extracted with 5% methanol-chloroform. Organic layer was washed with water, brine and dried over anh.sodium sulfate. Concentration of the solvent afforded 170mg of residue containing 5-ethoxy-9-hydroxycamptothecin and 5,9-dihydroxycamptothecin in the ratio of 3:1.

**Step 2:** Separation of the mixture by column chromatography using the solvent mixture of ethyl acetate-chloroform gave 75mg of 5-ethoxy-9-hydroxycamptothecin of the formula 1 along with 25mg of 9,5-dihydroxycamptothecin; mp: 230°C.; IR: 3400, 2920, 1745, 1663, 1597, 1360, 1280, 1228, 1157, 1083, 902, 816 cm⁻¹; ¹H NMR (CDCl₃): 6 8.83(s,1H), 7.78 (d, J=6.8Hz, 1H), 7.67-7.56 (m, 2H), 7.01(s, 0.5H), 6.98(s, 0.5H), 6.91(s, 0.5H), 6.81(s, 0.5H), 5.70(d, J=16Hz, 1H), 5.33(d, J=16Hz, 1H), 4.15-3.91(m, 2H), 1.90(m, 2H), 1.05(t, J=7Hz, 3H); Mass (m/z): 409(M+1), 364, 335, 320, 291, 267, 263, 221, 206, 171, 159, 129, 111, 98, 85.

### EXAMPLE 12

### Preparation of 9,5-Dihydroxycamptothecin of the formula 13 where R¹ = OH, R² =R³ = R⁴= R⁵ = H

**Step 1:** Initially 5-Ethoxy-9-hydroxycamptothecin of the formula 1 where R¹=OH, R²=R³=R⁴=R⁵=H, R⁶=Et, was prepared as described in the example 11.

**Step 2:** 25 ml of 80% HCl was added to 560mg of 5-ethoxy-9-hydroxy camptothecin of the formula 1 where R¹=OH, R²=R³=R⁴=R⁵=H, R⁶=Et, dissolved in 25ml of ethanol and heated to reflux for 16h. At the end, excess water and ethanol were removed as an azeotropic mixture and the residue was extracted with ethylacetate. Organic layer was washed with brine and dried over anh.sodium sulfate. Concentration of the solvent afforded 320mg of 9,5-dihydroxycamptothecin of the formula 13 after purification over silica gel column chromatography; mp: 102°C.; IR: 3400, 1744, 1659, 1594, 1462, 1361, 1280, 1229, 1049, 820 cm⁻¹: ¹H NMR (DMSO-d6): δ 10.82 (s, 1H, D₂O exchangeable), 7.63-7.69(m, 2H), 7.22(s, 0.5H), 7.19(s, 0.5H), 7.11(d, J=7Hz, 1H), 6.98(s, 0.5H), 6.95(s, 0.5H), 6.50(s, 1H, D₂O exchangeable), 5.42(s, 2H), 1.89(m, 2H), 0.90(t, J=7Hz, 3H); Mass(m/z): 380(M+1), 320, 305, 293, 264,

### EXAMPLE 13

### Preparation of 5-Ethoxy-9-hydroxy-7-ethylcamptothecin of the formula 1 where R¹=OH, R²=R³=R⁴=H, R⁵=R⁶=Et

**Step 1:** To a mixture of 9-hydroxy-7-ethylcamptothecin of the formula 2 where R¹=OH, R²=R³=R⁴=H R⁵=Et (150mg), ferric chloride (150mg), dissolved in 30ml of ethanol, 2ml of sulfuric acid was added dropwise and continued heating at 85°C. for 40h. Excess acid and ethanol were removed under vaccum and the residue was extracted with ethylacetate. Organic layer was washed with water, brine and dried over anh.sodium sulfate. Concentration of the solvent afforded 120mg of residue containing 5-ethoxy-9-hydroxy-7-ethylcamptothecin and 9,5-dihydroxy-7-ethylcamptothecin in the ratio of 5:1.

**Step 2:** Separation of the mixture by column chromatography using the solvent mixture of acetone-chloroform gave 85mg of 5-ethoxy-9-hydroxy-7-ethylcamptothecin of the formula 1 and 15mg of 5,9-dihydroxy-7-ethylcamptothecin; mp: 240°C.; IR; 3500, 2976, 1749, 1662, 1588, 1555, 1461, 1390, 1147, 1079, 921 cm⁻¹; ¹H NMR (DMSO-d6): δ 10.77(s, 1H D₂O exchangeable), 7.62-7.67(m, 2H), 7.57(d, J=8Hz, 1H), 7.08-7.18(m,2H), 6.50(s, 1H, D₂O exchangeable), 5.39(s, 2H), 4.08(m, 2H), 3.42(m, 2H), 1.87(m, 2H), 1.35(t, J=7Hz, 3H); 0.87(t, J=7Hz, 3H); Mass (m/z) : 437(M+1), 392, 363, 348, 333, 291, 261, 246, 219, 191, 149, 119, 89.

### EXAMPLE 14

### Preparation of 9,5-Dihydroxy-7-ethylcamptothecin of the formula 13 where R¹=OH, R²=R³=R⁴=H, R⁵=Et

**Step 1:** Initially 5-Ethoxy-9-hydroxy-7-ethylcamptothecin of the formula 1 where R¹=OH, R²=R³=R⁴=H, R⁵=R⁶=Et, was prepared as described in the example 13.

**Step 2:** 35 ml of 80% HCl was added to 560mg of 5-ethoxy-9-hydroxy-7-ethylcamptothecin of the formula 1 where R¹=OH, R²=R³=R⁴=H, R⁵=R⁶=Et, dissolved in 25ml of ethanol and heated to reflux for 16h. At the end, excess water and ethanol were removed as an azeotropic mixture and the residue was extracted with ethylacetate. Organic layer was washed with brine and dried over anh.sodium sulfate. Concentration of the solvent afforded 380mg of 5,9-dihydroxy-7-ethylcamptothecin of the formula 13 after purification over silica gel column chromatography; mp: 165°C.: IR 3351, 2929, 1744, 1657, 1606, 1460, 1218, 1162, 1035, 872 cm⁻¹: ¹H NMR (DMS0-d6) : δ 10.62 (s, 1H, D₂O exchangeble), 7.60-7.57(m, 2H) 7.16-7.00(m, 3H), 5.40(s, 2H, 3.42(q, J=7.6Hz, 2H), 2.08(m,2H), 1.33(t, J=7Hz, 3H), 0.89(t, J=7Hz, 3H); Mass(m/z): 408(M+1), 380, 336, 319, 291, 267, 235, 219, 185, 127, 99, 83.

### EXAMPLE 15

### Preparation of 9-nitro-5-ethoxy camptothecin of the formula 1 where R¹=NO₂, R²=R³=R⁴=R⁵=H, R⁶=Et

**Step 1:** To a mixture of 9-nitrocamptothecin of the formula 2 where R¹=NO₂ R²=R³=R⁴=R⁵=H, (1g), ferric chloride (1g), dissolved in 100ml of ethanol, 10ml of sulfuric acid was added dropwise and continued heating at 85°C. for 24h. Excess acid and ethanol were removed under vaccum and the residue was extracted with ethylacetate. Organic layer was washed with water, brine and dried over anh.sodium sulfate. Concentration of the solvent afforded 900mg of yellowish powder.

**Step 2:** Purification of the above solid material over silica gel column chromatography using acetone-chloroform solvent mixture as eluent furnished 700mg of 9-nitro-5-ethoxycamptothecin of the formula 1 and 80mg of 9-nitro-5-hydroxycamptothecin of the formula 13 where R¹=NO₂, R²=R³=R⁴=R⁵=H; mp: 202°C.; IR (KBr): 3474, 1743, 1668, 1622, 1526, 1344, 1154, 1073, 831cm⁻¹; ¹H NMR(CDCl₃, 200 MHz): δ 9.23 (s, 1H), 8.52(d, J=9Hz, 1H), 8.47(d, J=9Hz, 1H), 7.92(t, J=8.2Hz, 1H), 7.55(s, 1H), 6.91(s, 1H), 5.71(d, J=16Hz, 1H), 5.28(d, J=16Hz, 1H), 4.39-3.98(m, 2H), 3.75(br s, 1H, D₂O exchangeable), 1.99-1.79(m, 2H), 1.32(t, J=7Hz, 3H), 1.04(t, J=7Hz, 3H); Mass(m/z): 438(M+1), 407, 393, 364, 349, 319, 262, 118.

### EXAMPLE 16

### Preparation of 12-Nitro-5-ethoxycamptothecin of the formula 1 where R¹=R²=R³=R⁵=H, R⁴=NO₂, R⁶=Et

**Step 1:** To a mixture of 12-nitrocamptothecin of the formula 2 where R⁴=NO₂ R¹=R²=R³=R⁵=H, (2g), ferric chloride (2g), dissolved in 150ml of ethanol, 15ml of sulfuric acid was added dropwise and continued heating at 85°C. for 24h. Excess acid and ethanol were removed under vaccum and the residue was extracted with ethylacetate. Organic layer was washed with water, brine and dried over anh.sodium sulfate. Concentration of the solvent gave a gummy solid material.

**Step 2:** Purification of the above residue over silica gel column chromatography using acetone-chloroform solvent mixture as eluent afforded 1.4g of yellowish powder containing 12-nitro-5-ethoxycamptothecin of the formula 1 and 100mg of 12-nitro-5-hydroxycamptothecin of the formula 13 where R⁴=NO₂, R¹=R²=R³=R⁵=H; mp: 250°C.; IR (KBr): 3450, 1750, 1666, 1618, 1525, 1357, 1154, 1042, 766 cm⁻¹; ¹H NMR (CDCl₃, 200 MHz); δ 8.49(s, 1H), 8.17(d, J=9Hz, 1H), 8.14(d, J=9Hz, 1H), 7.75(t, J=8.2Hz, 1H), 7.54( s, 1H), 6.95(s, 0. 5H), 6.82(s, 0.5H), 5.71(d, J=16Hz, 1H), 5.26(d, J=16Hz, 1H), 4.31-3.91(m, 2H), 3.75(m, br s, 1H, D₂O exchangeable), 2.05-1.81(m, 2H), 1.35(1, J=7Hz, 3H), 1.05(1, J=7Hz, 3H); Mass (m/z) : 438(M+1), 420, 393, 376, 364, 349, 319, 84.

### EXAMPLE 17

### Preparation of 10-hydroxy-5-ethoxy camptothecin (known compound)

**Step 1:** To a mixture of 10-hydroxycamptothecin of the formula 2 where R²=OH, R¹=R³=R⁴=R⁵=H, (200mg), ferric chloride (200mg), dissolved in 10ml of ethanol, 1.5ml of sulfuric acid was added dropwise and continued heating at 85°C. for 24h. Excess acid and ethanol were removed under vaccum and the residue was extracted with 5% methanolethylacetate. Organic layer was washed with water, brine and dried over anh.sodium sulfate. Concentration of the solvent afforded yellow solid material.

**Step 2:** Purification of the above solid over silica gel column chromatography using acetone-chloroform solvent mixture as eluent provided 100mg of 10-hydroxy-5-ethoxycamptothecin of the formula 1 and 20mg of 10,5-dihydroxycamptothecin of the formula 13 where R²=OH, R¹=R³=R⁴=R⁵=H; mp; 165°C.; IR (KBr) ; 3384, 1747, 1662, 1608, 1229, 1044, 831 cm⁻¹; ¹H NMR(CDCl₃+ DMSO, 200MHz): δ 9.8(1H, br s, D₂O exchangeable), 8.25(s, 1E), 8.05(d, J=6Hz, 1H), 7.56-7.39(m, 2H), 7.25(s, 1H), 6.85(s, 0.5H), 6.70(s, 0.5H), 5.58(d, J=16Hz, 1H), 5.35 (d, J=16Hz, 0.5H), 5.21(d, J=16Hz, 0.5H), 4.35-3.75(m, 4H), 3.50( br s, 1H, D₂O exchangeable), 2.10-3.78 (m, 2H), 1.22 (t, J=7Hz, 3H), 1.05(t, J=7Hz, 3H); Mass (m/z): 409(M+1), 392, 364, 349, 335, 320, 291, 235, 117, 84.

### EXAMPLE 18

### Preparation of 10-Hydroxy-7-ethyl-5-ethoxy camptothecin (known compound)

**Step 1:** To a mixture of 10-hydroxy-7-ethylcamptothecin of the formula 2 where R²=OH, R¹=R³=R⁴=H, R⁵=Et, (200mg), ferric chloride (200mg), dissolved in 10ml of ethanol, 1.7ml of sulfuric acid was added dropwise and continued beating at 80°C. for 20h. Excess acid and ethanol were removed under vacuum and the residue was extracted with ethylacetate. Organic layer was washed with water, brine and dried over anh.sodium sulfate. Evaporation of the solvent gave a solid material.

**Step 2:** Purification of the above solid residue over silica gel column chromatography using acetone-chloroform solvent mixture as eluent afforded 85mg of 10-hydroxy-7-ethyl-5-ethoxy-camptothecin as yellowish powder and 20mg of 10,5-dihydroxy-7-ethylcamptothecin of the formula 13 where R²=OH, R¹=R³=R⁴=H, R⁵=Et; mp: 190°C.; IR (KBr): 3277, 1746, 1660, 1599, 1231, 1078, 800 cm⁻¹; ¹H NMR (CDCl₃+ DMSO): δ 9.6(br s, 1H, D₂O exchangeable), 8.01( d, J=8.7Hz, 1H), 7.51-7.35(m, 3H), 6.92(s, 0.5H), 6.80(s, 0.5H), 5.66(d, J=16Hz, 1H), 5.22(d, J=16Hz, 1H), 3.85-3.65(m, 2H), 3.35-2.95(m, 2H), 1.95-1.75(m, 2H), 1.37(t, J=7.4Hz, 3H), 1.17(t, J=7.2Hz, 3H), 0.99(t, J=7.4Hz, 3H); Mass (m/z): 437(M=1), 392, 363, 348, 333, 291, 147, 84.

### EXAMPLE 19

### Preparation of 9-amino-5-ethoxycamptothecin of the formula 1 where R¹=NH₂, R²=R³=R⁴=R⁵=H, R⁶=Et

**Step 1:** To a mixture of 9-aminocamptothecin of the formula 2 where R¹=NH₂ R²=R³=R⁴=R⁵=H, (120mg), ferric chloride (112mg), dissolved in 10ml of ethanol, 1.5ml of ethereal borontrifluoride(BF₃-Et₂O) was added dropwise and continued heating at 80°C. for 16h. Ethanol was removed under vaccum and the residue was extracted with ethylacetate. Organic layer was washed with water, brine and dried over anh.sodium sulfate which upon evaporation of the solvent gave a thick gummy solid.

**Step 2:** Purification of the above residue over silica gel column chromatography using acetone-chloroform solvent mixture as eluent afforded 65mg of 9-amino-5-ethoxycamptothecin of the formula 1; mp: 170°C.; IR 3221, 1744, 1661, 1231, 1157, 1074, 815 cm⁻¹: ¹H NMR (CDCl₃+DMSO-d₆): 6 8.69(s, 1H), 7.64(s, 1H), 7.63-7.51(m,2H), 7.06(d, J=5.41Hz, 1H), 6.90(s, 0.5H), 6.80(s, 0.5H), 5.65(d, J=16Hz, 1H), 5.26(d, J=16Hz, 1H), 4.19-3.98(m, 1H), 3.97-3.78(m, 1H), 2.98(br s, 3H, D₂O exchangeable), 1.95-1.80(m, 2H), 1.39-1.19(m, 3H), 1.11-0.95(m, 3H) ; Mass(m/z): 407 (M+2), 389, 363, 334, 319, 290, 262, 233, 101.

### EXAMPLE 20

### Preparation of 9-amino-5-methoxycamptothecin of the formula 1 where R¹=NH₂, R²=R³=R⁴=R⁵=H, R⁶=Me

**Step 1:** To a mixture of 9-aminocamptothecin of the formula 2 where R¹=R²=R³=R⁴=R⁵=H, (180mg), ferric chloride (162mg), dissolved in 15ml of methanol, 2ml of ethereal borontrifluoride(BF₃-Et₂O) was added dropwise and continued heating at 80°C. for 16h. Methanol was removed under vacuum and the residue was extracted with ethylacetate. Organic layer was washed with water, brine and dried over anh.sodium sulfate which upon evaporation of the solvent gave a thick gummy solid.

**Step 2:** Purification of the above residue over silica gel column chromatography using acetone-chloroform solvent mixture as eluent afforded 125mg of 9-amino-5-methoxycamptothecin of the formula 1; mp: 200°C.; IR: 3364, 2925, 1744, 1660, 1610, 1156, 1081, 811 cm⁻¹; ¹H NMR (CDCl₃+ DMS0-d6): δ 8.82 (s, 1H), 7.60(s, 1H), 7.63-7.46(m, 2H), 6.97(d, J=7Hz, 1H), 6.89(s, 0.5H, 6.80(s, 0.5H), 5.6(d, J=16Hz, 1H), 5.25(d, J=16Hz, 1H), 3.57(s, 1.5H), 3.46(s, 1.5H), 3.41(br s, 1H, D₂O exchangeable), 3.15(br s, 2H, D₂O exchangeable), 2.05-1.89(m,2H), 1.01(t, J=7Hz, 3H); Mass(m/z): 393, (M+1), 376, 363, 349, 334, 319, 290, 262, 233, 205, 116.

### EXAMPLE 21

### Preparation of 9-Nitro-5-hydroxy camptothecin of the formula 13 where R¹=NO₂, R²=R³=R⁴=R⁵=H

**Step 1:** Initially 9-nitro-5-ethoxycamptothecin of the formula 1 where R¹=NO₂, R²=R³=R⁴=R⁵=H, R⁶=Et was prepared as described in the example 15.

**Step 2:** 80 ml of 50% HCl was added to 1.0g of 9-nitro-5-ethoxycampothecin of the formula 1 where R¹=NO₂, R²=R³=R⁴=R⁵=H, R⁶=Et, dissolved in 20ml of ethanol and heated to reflux for 30h. At the end, excess water and ethanol were removed as an azeotropic mixture and the residue was extracted with ethylacetate. Organic layer was washed with brine and dried over anh.sodium sulfate. Concentration of the solvent afforded 700mg of 9-nitro-5-hydroxycamptothecin of the formula 13 after purification over silica gel column chromatography.; mp: 278°C.; IR (KBr) : 3402, 1744, 1657, 1602, 1533, 1155, 1051, 833 cm⁻¹; ¹H NMR (CDCl₃ +DMSO, 200MHz): δ 9.28(s, 1H), 8.50(d, J=8.6Hz, 1H), 8.45(d, J=8.6Hz, 1H), 7.96(t, J=8.2Hz, 1H), 7.59(s, 0.5H), 7.58(s, 0.5H), 7.12(s, 0.5H), 7.08(s, 0.5H), 5.67(d, J=16Hz, 1H), 5.27(d, J=16Hz, 1H), 1.92(q, J=7.2Hz, 2H), 1.07(t, J=7Hz, 3H).

### EXAMPLE 22

### Preparation of 10,5-Dihydroxy camptothecin (known compound)

**Step 1:** Initially 10-Hydroxy-5-ethoxycamptothecin of the formula 1 where R²=OH, R¹=R³=R⁴=R⁵=H, R⁶=Et was prepared as described in the example 17.

**Step 2:** 10 ml of 50% HCl was added to 250mg of 10-hydroxy-5-ethoxycamptothecin of the formula 1 where R²=OH, R¹=R³=R⁴=R⁵=H, R⁶-Et, dissolved in 10ml of ethanol and heated to reflux for 20h. At the end, excess water and ethanol were removed as an azeotropic mixture and the residue was extracted with ethylacetate. Organic layer was washed with brine and dried over anh.sodium sulfate. Concentration of the solvent afforded 210mg of 10,5-dihydroxycamptothecin of the formula 13 after purification over silica gel column chromatography.; mp: 240°C.; IR (KBr): 3226, 1743, 1659, 1596, 1382, 1231, 1048, 832 cm⁻¹; ¹H NMR (CDCl₃+DMSO): δ 10.0 (br s, 1H, D₂O exchangeable), 8.31(s, 0.5H), 8.29(s, 0.5H), 8.05(d, J=6Hz, 0.5H), 7.95(d, J=6Hz, 0.5H), 7.95 (d, J=6Hz, 0.5H), 7.50-7.31(m, 2H), 7.21(s, 1H), 6.95(s, 0.5H), 6.85(s, 0.5H), 5.55(d, J=16Hz, 1H), 5.25(d, J=16Hz, 1H), 3.99(br s, 1H, D₂O exchangeable), 2.05-1.81 (m, 2H), 1.0 (t, J=7Hz, 3H); Mass (m/z): 381(M+1), 352, 336, 320, 264, 149, 83.

### EXAMPLE 23

### Preparation of 12-Nitro-5-hydroxy camptothecin of the formula 13 where R¹=R²=R³=R⁵=H, R⁴=NO₂

**Step 1:** Initially 12-nitro-5-ethoxycamptothecin of the formula 1 where R⁴=NO₂, R¹=R²=R³=R⁵=H, R⁶=Et was prepared as described in the example 16.

**Step 2:** 125 ml of 50% HCl was added to 2g of 12-nitro-5-ethoxycamptothecin of the formula 1 where R⁴=NO₂, R¹=R²=R³=R⁵=H, R⁶=Et, dissolved in 30ml of ethanol and heated to reflux for 24h. At the end, excess water and ethanol were removed as an azeotropic mixture and the residue was extracted with ethylacetate. Organic layer was washed with brine and dried over anh.sodium sulfate. Concentration of the solvent afforded 1.5g of 12-nitro-5-hydroxycamptothecin of the formula 13 after purification over silica gel column chromatography; mp: 247°C.: IR (KBr): 3371, 1746, 1664, 1602, 1532, 1380, 1048, 829 cm⁻¹: ¹H NMR (CDCl₃, 200MHz) : δ 8.58(s, 1H), 8.17(d, J=9Hz, 1H), 8.12(d, J=9Hz, 1H), 7.74(t, J=8.2Hz, 1H), 7.58(s, 1H), 7.12(s, 0.5H), 7.08(s, 0.5H), 5.71(d, J=16Hz, 1H), 5.26(d, J=16Hz, 1H), 3.90(br s, 1H, D₂O exchangeable), 1.99-1.85(m, 2H), 1.05(t, J=7Hz, 3H); Mass (m/z) : 409(M+1), 393, 380, 363, 348, 333, 318, 149, 85.

### EXAMPLE 24

### Preparation of 5-(2'-Chloroethoxy)camptothecin of the formula 1 where R¹=R²=R³=R⁴=R⁵=H, R⁶=CH₂CH₂Cl

**Step 1:** To a mixture of 20(S)-Camptothecin of the formula 3 (1g), ferric chloride (1g), dissolved in 25ml of 2-chloroethanol 5ml of sulfuric acid was added dropwise and continued heating at 90°C. for 24h. Excess acid and 2-chloroethanol were removed under vacuum and the residue was extracted with ethylacetate. Organic layer was washed with water, brine and dried over anh.sodium sulfate. Concentration of the solvent afforded 1.2g of brownish solid

**Step 2:** The above solid was purified by column chromatography to afford 650mg of 5-(2'-chloroethoxy)camptothecin of the formula 1 and 150mg of previously prepared 5-hydroxy-camptothecin of the formula 13 where R¹=R²=R³=R⁴=R⁵=H; mp :202°C.; [α]_{D} at 28°C.=+5.37 (c 0.093, CHCl₃); IR: 3354, 1744, 1662, 1622, 1223, 1160, 1090, 1044, 752, 663 cm⁻¹; Partial ¹H NMR data in CDCl₃: δ 6.92 (s, 0.5H), 6.82(s, 0.5H), 4.51 (t, J=5Hz, 1.5H), 4.38(t, J=5Hz, 1.5H), 3.75(s, 1H, D₂O exchangeable), 3.85-3.58(m, 2H), 2.00-1.78(m, 2H), 1.06(t, J=7.5Hz, 3H); Mass(m/z): 426(M+1), 391, 377, 363, 348, 319, 105, 84, 51.

### EXAMPLE 25

### Preparation of 5-trifluoroethoxycamptothecin of the formula 1 where R¹=R²=R³=R⁴=R⁵=H, R⁶=CH₂CF₃

**Step 1:** To a mixture of 20(S)-Camptothecin of the formula 3 (0.5g), ferric chloride (0.5g), dissolved in 18ml of 2,2,2-trifluoroethanol, sulfuric acid was added dropwise and continued heating at 80°C. for 24h. Excess acid and trifluoroethanol were removed under vacuum and the residue was extracted with ethylacetate. Organic layer was washed with water, brine and dried over anh.sodium sulfate. Concentration of the solvent afforded 600mg of solid material.

**Step 2:** The above solid material was purified by column chromatography to give 250mg of 5-trifluoroethoxycamptothecin of the formula 1 along with 150mg of previously prepared 5-hydroxycamptothecin of the formula 13; mp 188°C. ; IR: 3438, 1748, 1667, 1620, 1160, 1106, 1003 cm⁻¹; Partial ¹H NMR data in CDCl₃: δ 6.84 (s, 0.5H), 6.75(s, 0.5H), 5.21-4.90(m, 1H), 4.60-4.38(m,2H), 3.70(s, 1H, D₂O exchangeable), 2.0-1.79(m, 2H), 1.15-0.99(m, 3H); Mass (m/z):447 (M+1), 378, 348, 304, 111, 69.

### EXAMPLE 26

### Preparation of 5-(2'-Hydroxyethoxy)camptothecin of the formula 1 where R¹=R²=R³=R⁴=R⁵=H, R⁶=CH₂CH₂OH

**Step 1:** To a mixture of 20(S)-Camptothecin of the formula 3 (1g), ferric chloride (1g), dissolved in 10ml of ethylene glycol, 5ml of sulfuric acid was added dropwise and continued heating at 70°C. for 36h. Excess acid and ethylene glycol were removed under vacuum and the residue was extracted with ethylacetate. Organic layer was washed with water, brine and dried over anh.sodium sulfate. Concentration of the solvent gave 1.1g of yellowish powder.

**Step 2:** The solid obtained as above was subjected to column purification using ethylacetate-hexane solvent mixture to afford 700mg of 5-(2'-Hydroxyethoxy)campto-thecin of the formula 1 and 200mg of previously prepared 5-hydroxycamptothecin of the formula 13 where R¹=R²=R³=R⁴=R⁵=H; mp: 190°C; [α]_{D} at 26°C. = + 28.30 (c 0.106, CHCl₃); IR: 3300, 3285, 1745, 1665, 1620, 1605, 1227, 1160, 1112, 1047 cm⁻¹; Partial ¹H NMR data in CDCl₃: δ 7.01 (s, 0.5H), 6.92 (s, 0.5H), 4.30-3.71(m, 4H), 3.75( br s, 2H, D₂O exchangeable), 2.0-1.79(m 2H), 1.15-0.95 (m, 3H); Mass (m/z): 408(M+1), 390, 378, 364, 348, 319, 101, 76.

### EXAMPLE 27

### Preparation of 10-Hydroxy-5-trifluoroethoxycamptothecin of the formula 1 where R¹=R³=R⁴=R⁵=H, R2=OH, R⁶=CH₂CF₃

**Step 1:** Initially 10,5-dihydroxycamptothecin of the formula 13 where R¹=R³=R⁴=R⁵=H, R²=OH, was prepared as described in the example 22.

**Step 2:** A mixture of 10,5-dihydroxycamptothecin of the formula 13 where R²=OH, R¹=R³=R⁴=R⁵=H (200mg) and trifluoroethanol (1mL) were suspended in 50ml of dichloroethane and heated to reflux in the presence of sulfuric acid (0.5ml) for 18h. Reaction mixture was concentrated to dryness and the residue was extracted with ethylacetate. Organic layer was washed with water and brine and dried over anh.sodium sulfate. Evaporation of the solvent furnished an oily residue which was purified over silica gel column using acetonechloroform as an eluent to get 140mg of 10-Hydroxy-5-trifluoroethoxycamptothecin of the formula 1 as a solid; mp: 237°C.; IR: 3420, 1748, 1664, 1605, 1159 cm⁻¹; ¹H NMR(DMSO-d6): δ 10.48(s, 1H, D₂O exchangeable), 8.45(s, 1H), 8.04(d, J=9Hz, 1H), 7.47(d, J=9Hz, 1H), 7.40(s, 1H), 7.18(s, 1H), 7.11(s, 0.5H), 7.06(s, 0.5H), 6.58(s, 1H, D₂O exchangeable), 5.41(s,2H), 5.05-4.55(m, 2H), 2.05-1.75(m,2H), 1.00-0.8(m,3H); ¹³C NMR (DMSO-d6) : δ 172.4, 161.0, 157.7, 157.1, 151.2, 147.5, 144.3, 143.7, 131.0, 130.8, 129.8, 129.1, 124.0, 121.4, 120.7, 109.6, 96.6, 89.7, 72.3, 65.1, 30.4, 7.8: Mass(m/z): 462(M+1), 418, 364, 320, 291, 263.

### EXAMPLE 28

### Preparation of 9-Nitro-5-trifluoroethoxycamptothecin of the formula 1 where R²=R³=R⁴=R⁵=H, R¹=NO₂ R⁶=CH₂CF₃

**Step 1:** Initially 9-nitro-5-hydroxycamptothecin of the formula 13 where R²=R³=R⁴=R⁵=H, R¹=NO₂ was prepared as described in the example 21.

**Step 2:** A mixture of 9-nitro-5-dihydroxycamptothecin of the formula 13 where R¹=NO₂, R²=R³=R⁴=R⁵=H (100mg) and trifluoroethanol (0.5mL) were suspended in 25ml of dichloroethane and heated to reflux in the presence of sulfuric acid (0.3ml) for 18h. Reaction mixture was concentrated to dryness and the residue was extracted with ethylacetate. Organic layer was washed with water and brine and dried over anh.sodium sulfate. Evaporation of the solvent furnished an oily residue which was purified over silica gel column using acetone-chloroform as an eluent to get 60mg of 9-nitro-5-trifluoroethoxycamptothecin of the formula 1 as a solid.; mp 210°C.; IR: 3457, 1745, 1665, 1623, 1527, 1154, 1000 cm⁻¹; ¹H NMR (CDCl₃): δ 9.30(s, 1H), 8.53(d, J=8.6Hz, 1H), 8.49(d, J=8.6Hz, 1H), 7.94(t, J=8Hz, 1H) , 7.62(s, 0.5H), 7.60(s, 0.5H), 6.87(s, 0.5H), 6.81(s, 0.5H), 5.69(d, J=16Hz, 1H), 5.29(d, J=16Hz, 1H), 4.97(m, 1H), 4.52(m, 1H), 3.90(br s,1H, D₂O exchangeable), 1.90(m,2H), 1.05(t, J=7Hz, 3H); Mass (m/z): 491(M+1), 461, 446, 418, 393, 364, 349, 319, 290, 216

### EXAMPLE 29

### Preparation of 5-(2'-fluoroethoxy)camptothecin of the formula 1 where R¹=R²=R³=R⁴=R⁵=H, R⁶=CH₂CH₂F

**Step 1:** Initially 5-hydroxycamptothecin of the formula 13 where R¹=R²=R³=R⁴=R⁵=H, was prepared as described in the example 2.

**Step 2:** A mixture of 5-hydroxycamptothecin of the formula 13 where R¹=R²=R³=R⁴=R⁵=H (200mg) and 2-fluoroethanol (2mL) were suspended in 30ml of dichloroethane and heated to reflux in the presence of sulfuric acid (0.3ml) for 18h. Reaction mixture was concentrated to dryness and the residue was extracted with ethylacetate. Organic layer was washed with water and brine and dried over anh.sodium sulfate. Evaporation of the solvent furnished an oily residue which was purified over silica gel column using acetone-chloroform as an eluent to get 130mg of 5-(2'-fluoroethoxy)camptothecin of the formula 1 as a solid; mp: 174°C.; IR: 1745, 1664, 1615, 1160, 1040, 752 cm⁻¹; ¹N NMR (CDCl₃ + DMSO-d6): δ 8.46(s, 1H), 8.20(d, J=8Hz, 1H ), 7.95(d, J=8Hz, 1H), 7.83 (t, 3= 6.8Hz, 1H), 7.65-7.55(m, 2H), 6.86(s, 0.5H), 6.78(s, 0.5H), 5.68(d, J=16.5Hz, 1H), 5.26(d, J=16.5Hz, 1H), 4.90-4.20 (m, 4H), 4.44(s, 1H, D₂O exchangeable), 2.05-1.85(m, 2H), 1.12-0.95(m, 3H) ; Mass(m/z): 410(M+1), 365, 348, 319, 304.

### EXAMPLE 30

### Preparation of 10-Hydroxy-5-(2'-fluoroethoxy)-camptothecin of the formula 1 where R¹=R³=R⁴=R⁵=H, R²=OH, R⁶=CH₂CH₂F

**Step 1:** Initially 10,5-dihydroxycamptothecin of the formula 13 where R¹=R³=R⁴=R⁵=H, R²=OH, was prepared as described in the example 22.

**Step 2:** A mixture of 10,5-dihydroxycamptothecin of the formula 13 where R²=OH, R¹=R³=R⁴=R⁵=H (100mg) and 2-fluoroethanol (2mL) were suspended in 25ml of dichloroethane and heated to reflux in the presence of sulfuric acid (0.2ml) for 16h. Reaction mixture was concentrated to dryness,and the residue was extracted with ethylacetate. Organic layer was washed with water and brine and dried over anh.sodium sulfate. Evaporation of the solvent furnished an oily residue which was purified over silica gel column using acetone-chloroform as an eluent to get 60mg of 10-Hydroxy-5-fluoroethoxycamptothecin of the formula 1 as a solid; mp: 258- 260°C.; IR: 3225, 1748, 1660, 1593, 1159 cm⁻¹; ¹H NMR (CDCl₃ + DMSO-d6): δ 10.0 (br s, 1H, D₂O exchangeable), 8.31(s, 1H), 8.00(d, J=6Hz, 1H), 7.80(s, 1H), 7.45(d, J=6Hz, 1H), 7.40(s, 1H), 6.85(s, 0.5H), 6.80(s, 0.5H), 6.15(s, 1H, D₂O exchangeable), 5.55(d, J=16Hz, 1H), 5.23(d, J=16Hz, 1H), 4.85-4.20(m, 4H), 2.05-1.81(m,2H), 1.0(t, J=7Hz, 3H); Mass(m/z): 426(M+1), 382, 364, 320.

### EXAMPLE 31

### Preparation of 5-(2'-Fluoroethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 80mg of 5-hydroxy-7-ethylcamptothecin and 0.1ml of p-toluenesulfonic acid suspended in 12ml of benzene, 20mg of 2-fluoroethanol was added and heated the mixture to reflux temperature for 14h. Reaction was quenched with a drop of pyridine and extracted with ethyl acetate. Organic layer was washed with water, NaHCO₃, brine and concentrated to dryness. The residue was purified by silica gel column chromatography using ethyl acetate-chloroform as eluent to afford 60mg of 5-(2'-fluoroethoxy)-7-ethylcampto-thecin; mp: 112°C.; IR: 3070, 1748, 1665, 1605, 1456, 1155, 1038, 767 cm⁻¹; ¹H NMR (CDCl₃): δ 8.21 (d, J=9.2Hz, 1H), 8.17(d, J=9.2Hz, 1H), 7.82(t, J=7.4Hz, 1h), 7.67(t, J=7.4Hz, 1H), 7.57(s, 0.5H), 7.54(s, 0.5H), 7.00(s, 0.5H), 6.89(s, 0.5H), 5.69(d, J=16Hz, 1H), 5.27(d, J=16Hz, 1H), 4.81-4.12(m, 4H), 3.51-3.15(m, 2H), 1.93(m, 2H), 1.45(t, J=7Hz, 3H), 1.05(m, 3H); Mass(m/z): 438(M+1), 420, 406, 376, 347, 332, 317, 245, 91.

### EXAMPLE 32

### Preparation of 5-(2'-Hydroxyethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 250mg of 5-hydroxy-7-ethylcamptothecin and 10 µl of conc.sulfuric acid suspended in 25ml of dichloroethane, 0.5ml of ethylene glycol was added and heated the mixture to reflux temperature for 14h. Reaction was quenched with a drop of pyridine and extracted with ethyl acetate. Organic layer was washed with water and brine and concentrated to dryness. The residue was purified by silica gel column chromatography using ethyl acetate-chloroform as eluent to furnish 180mg of 5-(2'-hydroxyethoxy)-7-ethylcamptothecin and 25mg of starting material; ¹H NMR (CDCl₃, 200MHz): δ 8.20(d, J=8Hz, 1H), 8.15(d, J=8Hz, 1H), 7.85(t, J=6.8Hz, 1H), 7.69(t, 7.3Hz, 1H), 7.56(s, 0.5H), 7.54(s, 0.5H), 7.11(s, 0.5H), 6.99(s, 0.5H), 5.72(d, J=16.5Hz, 1H), 5.28(d, J=16.5Hz, 0.5H), 5.26(d, J=16.5Hz, 0.5H), 3.95-3.65(m, 4H), 3.78 (br s, 2H, D₂O exchangeable), 3.5-3.18 (m, 2H), 1.95-1.81(m, 2H), 1.45(t, J=7.5Hz, 3H), 1.06(m, 3H).

### EXAMPLE 33

### Preparation of 5-(2'-Hydroxyethoxy)-10-hydroxycamptothecin

**Step 1:** Initially 10,5-dihydroxycamptothecin of the formula 13 where R¹=R³=R⁴=R⁵=H, R²=OH, was prepared as described in the example 22.

**Step 2:** To a mixture of 60mg of 10,5-dihydroxycamptothecin and 5mg of p-toluenesulfonic acid suspended in 10ml of dichloroethane, 25mg of ethylene glycol was added and heated the mixture to reflux temperature for 16h. Reaction was quenched with a drop of pyridine and extracted with ethyl acetate. Organic layer was washed with water and brine and concentrated to dryness. The residue was purified by silica gel column chromatography using methanol-chloroform as eluent to furnish 40mg of 5-(2'-hydroxyethoxy)-10-hydroxycampto-thecin and 10mg of starting material; IR; 3070, 1760, 1660, 1600, 1558, 1509, 1384, 1160, 1047, 832 cm⁻¹; ¹H NMR CDCl₃ + DMSO): δ 10.05(br, 1H D₂O exchangeable), 8.35(s, 1H), 8.05(d, J=9Hz, 1H), 7.75(s, 1H), 7.45(d, J=9Hz, 1H), 7.28(s, 1H), 6.95(s, 0.5H), 6.85(s, 0.5H), 5.65(d, J=16Hz, 1H), 5.25(d, J=16Hz, 1H), 4.11(m, 2H), 3.78(m, 2H), 4.05(br s, 1H, D₂O exchangeable), 1.98(m, 2H), 1.05(t, J=7Hz, 3H); Mass (m/z): 425, 408, 380, 364, 336, 320, 305, 264, 235, 147, 105.

### EXAMPLE 34

### Preparation of 5,10-Dihydroxy-7-ethylcamptothecin

**Step 1:** Initially 5-ethoxy-10-hydroxy-7-ethylcamptothecin of the formula 1 where R¹=R³=R⁴=H, R⁵=R⁶=Et, R²=OH was prepared as described in the Example 18.

**Step 2:** 12ml of 25%HCl was added to 200mg of 5-ethoxy-10-hydroxy-7-ethylcamptothecin dissolved in 10ml of ethanol and heated to reflux for 24h. Excess acid and ethanol were distilled off and the remaining residue was diluted with ethylacetate. The organic layer was washed with 5% NaHCO₃ solution and brine.
Concentration of the solvent and purification of the solid material over silica gel column using acetone-chloroform solvent mixture as eluent afforded 105mg of 5, 10-dihydroxy-7-ethylcamptothecin; mp : 197°C.; IR: 3268, 2975, 1748, 1656, 1597, 1514, 1230, 1161, 1052, 841 cm⁻¹; ¹H NMR (CDCl₃+DMSO): δ 10.0(br s, 1H, D₂O exchangeable), 8.05(d, J=9Hz, 1H), 7.76(s, 2H), 7.42(m, 1H), 7.04(s, 0.5H), 6.98(s, 0.5H), 5.65(d, J16Hz, 1H), 5.23(d, J=16Hz,1H), 3.51(br s, 1H, D₂O exchangeable), 3.45-3.12(m, 2H), 1.94(m, 2H), 1.42(t, J=7Hz, 3H), 1.01(t, J=7Hz, 3H); Mass(m/z): 408(M+1), 379, 364, 347, 335, 285, 169, 119, 101, 83.

### EXAMPLE 35

### Preparation of 5-(2'Hydroxyethoxy)-10-hydroxy-7-ethylcamptothecin

**Step 1:** Initially 7-ethyl-5,10-dihydroxy camptothecin of the formula 13 where R¹=R³=R⁴=H, R²=OH, R⁵=Et, was prepared as described in the example 34.

**Step 2:** To a mixture of 100mg of 10,5-dihydroxy-7-ethylcamptothecin and 5mg of p-toluenesulfonic acid suspended in 10ml of dichloroethane, 50mg of ethylene glycol was added and heated the mixture to reflux temperature for 16h. Reaction was quenched with a drop of pyridine and extracted with ethyl acetate. Organic layer was washed with water and brine and concentrated to dryness. The residue was purified by silica gel column chromatography using methanolchloroform as eluent to furnish 60mg of 5-(2'-hydroxyethoxy)-10-hydroxy-7-ethylcamptothecin and 12mg of starting material.; mp: 124°C.; ¹H NMR (CDCl₃+DMSO): δ 10.0 (br s, 1H, D₂O exchangeable), 8.02(d, J=9Hz, 1H), 7.55-7.39(m, 3H), 7.02(s, 0.5H), 6.93(s, 0.5H), 6.05 (br s, 1H, D₂O exchangeable), 5.63(d, J=16Hz, 1H), 5.23(d, J=16Hz, 1H), 3.94-3.54(m,2H), 3.41-3.05(m 2H), 1.93 (m,2H), 1.40(t, J=7Hz,3H), 1.02(m,3H); Mass (m/z) : 408(M+1), 379, 364, 347, 335, 285, 169, 119, 101, 83.

### EXAMPLE 36

### Preparation of 5-(2'-aminoethoxy) camptothecin

**Step 1:** Initially 5-hydroxy camptothecin of the formula 13 where R¹=R²=R³=R⁴=R⁵=H, was prepared as described in the example 2.

**Step 2:** To a mixture of 60mg of 5-hydroxycamptothecin and 5mg of p-toluenesulfonic acid suspended in 10ml of benzene, 15mg of 2-aminoethanol was added and heated the cure to reflux temperature for 14h. Reaction was quenched with a drop of pyridine and extracted with ethyl acetate. Organic layer was washed with water,

NaHCO₃, brine and concentrated to dryness. The residue was purified by silica gel column chromatography using methanolchloroform as eluent to furnish 36mg of 5-(2'-aminoethoxy)camptothecin and 10mg of starting material; mp: 170°C.; IR: 3451, 1740, 1664, 1604, 1383, 1189, 1042 cm⁻¹; Partial ¹H NMR data in (CDCl₃ + DMSO-d6) : 6 7.5(d, D₂O exchangeable, 1H), 7.15(d, D₂O exchangeable, 1H), 7.02 (s, 0.5H), 6.92(s, 0.5H), 5.65(d, J=16Hz, 1H), 5.28(d, J=16Hz, 1H), 4.24-3.85(m, 2H), 2.35(s, D₂O exchangeable, 1H), 2.34(m, 1H), 2.15-1.85(m, 3H), 1.12-0.95(m, 3H); Mass (m/z): 408(M+1), 364, 347, 319, 305, 291, 249, 103, 62.

### EXAMPLE 37

### Preparation of 5-(2'-aminoethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 85mg of 7-ethyl-5-hydroxycamptothecin and 5mg of p-toluenesulfonic acid suspended in 20ml of benzene, 11 mg of 2-aminoethanol was added and heated the mixture to reflux temperature for 10h. Reaction was quenched with a drop of pyridine and extracted with ethyl acetate. Organic layer was washed with water, NaHCO₃, brine and concentrated to dryness. The residue was purified by silica gel column chromatography using methanol-chloroform as eluent to afford 65mg of 5-(2'-aminoethoxy)-7-ethylcamptothecin. mp: 230°C.; Partial ¹H NMR in (CDCl₃+ DMSO-d6): δ 7.5(d, D₂O exchangeable, 1H), 7.15(d, D₂O exchangeable, 1H), 7.02(s, 0.5H), 6.92(s, 0.5H), 5.65(d, J=16Hz, 1H), 5.28(d, J=16Hz, 1H), 4.24-3.85(m, 2H), 2.35(s, D₂O exchangeable, 1H), 2.34(m, 1H), 2.15-1.85(m, 3H), 1.12-0.95(m, 1H); Mass (m/z): 408(M+1), 364, 347, 319, 305, 103, 74, 62.

### EXAMPLE 38

### Preparation of 5-(3'-dimethylaminopropoxy)-7-ethylcamptothecin

**Step 1**: Initially 5-hydroxy-7-ethylcamptothecin of the formula 13 where R¹=R²=R³=R⁴=H, R⁵=Et, was prepared as described in the example 4.

**Step 2**: To a mixture of 50mg of 7-ethyl-5-hydroxycamptothecin and 0.05ml of sulfuric acid suspended in 20ml of benzene, 30mg of 3-dimethylamino-1-propanol was added and heated the mixture to reflux temperature for 12h. Reaction was quenched with a drop of pyridine and extracted with ethyl acetate. Organic layer was washed with water, NaHCO₃, brine and concentrated to dryness. The residue was purified by silica gel column chromatography using methanol-chloroform as eluent to obtain 42mg of 5-(3'-dimethylaminopropoxy)-7-ethylcamptothecin; mp: 113°C.; Partial ¹H NMR data in (CDCl₃+ DMSO-d6): δ 6.95 (s, 0.5H), 6.85(s, 0.5H), 5.65(d, J=16Hz, 1H), 5.35(d, J=16Hz, 0.5H), 5.25(d, J=16Hz, 0.5H), 3.95-3.57(m, 2H), 3.30-3.05(m, 2H), 2.85(s, 3H), 2.83(s, 3H), 2.15-1.72(m, 6H), 1.45(t, J=7.5Hz, 3H), 1.12-0.95(m, 3H); Mass (m/z): 478(M+1), 434, 375, 347, 331, 169, 102, 84; Mass(m/z) : 478(M+1), 434, 375, 347, 331, 169, 102, 84.

### EXAMPLE 39

### Preparation of 5-(2'-N-pyrrolidinoethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 100mg of 7-ethyl-5-hydroxycamptothecin and 10mg of camphorsulfonic acid suspended in 25ml of benzene, 30mg of 1-(2-hydroxyethyl) pyrrolidine was added and heated the mixture to reflux temperature for 16h. Reaction was quenched with a drop of pyridine and extracted with ethyl acetate. Organic layer was washed with water, NaHCO₃, brine and concentrated to dryness. The residue was purified by silica gel column chromatography using methanol-chloroform as eluent to acetate 85mg of 5-(2'-N-pyrrolidinoethoxy)-7-ethylcamptothecin; mp: 225°C.; IR: 3424, 1749, 1666, 1616, 1384, 1156, 1078, 1049 cm-1; Partial ¹H NMR data in CDCl₃: d 7.02 (s, 0.5H), 6.95(s, 0.5H), 5.70(d, J=16Hz, 1H), 5.33(d, J=16Hz, 0.5H), 5.26(d, J=16Hz, 0.5H), 4.18-3.88(m, 2H), 3.45-3.15(m,2H), 3.06-2.58(m, 6H), 2.05-1.72(m, 6H), 1.43(t, J=8Hz, 3H), 1.15-0.95(m, 3H); Mass (m/z): 446 (M+1); 375, 347, 331, 245, 169, 116, 97, 84.

### EXAMPLE 40

### Preparation of 5-(2'-chloroethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 500mg of 7-ethyl-5-hydroxycamptothecin and 0.1ml of conc.sulfuric acid suspended in 30ml of benzene, 700mg of 2-chloroethanol was added and heated the mixture to reflux temperature using Dean-Stork apparatus for 8h. Reaction was quenched with a drop of pyridine and extracted with ethyl acetate. Organic layer was washed with water, NaHCO₃, brine and concentrated to dryness. The residue was purified by silica gel column chromatography using ethyl acetate-chloroform as eluent to provide 400mg of 5-(2'-chloroethoxy)-7-ethylcamptothecin; mp: 168°C.; ¹H NMR (CDCl₃, 200MHz): δ 8.20(d, J=9.5Hz, 1H), 8.15(d, J=9.5Hz, 1H), 7.82(t, J=8Hz, 1H), 7.67(t, J=8Hz, 1H), 7.54(d, 6.2Hz, 1H), 7.02(s, 0.5H), 6.90(s, 0. 5H), 5.70(d, J=16Hz, 0.5H), 5.69(d, J=16Hz, 0.5H), 5.26(d, J=16Hz, 0.5H), 5.25(d, J=16Hz, 0.5H), 4.61-3.95(m, 2H), 3.78-3.58(m, 2H), 3.50-3.15(m, 2H), 1.98-1.78(m, 2H), 1.45-(t, 3=7. 5Hz, 3H), 1.12-0.95(m, 3H); Mass (m/z): 455(M+1), 437, 409, 392, 376, 347, 331, 245, 115, 81.

### EXAMPLE 41

### Preparation of 5-(2'-dimethylaminoethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 100mg of 7-ethyl-5-hydroxycamptothecin and 0.1ml of conc.sulfuric acid suspended in 10ml of benzene, 50mg of 2-N,N-dimethylaminoethanol was added and heated the mixture to reflux temperature using Dean-Stark apparatus for 10h. Reaction was quenched with a drop of pyridine and extracted with ethyl acetate. Organic layer was washed with water, NaHCO₃, brine and concentrated to dryness. The residue was purified by silica gel column chromatography using methanol-chloroform as eluent to get 65mg of 5-(2'-dimethylaminoethoxy)-7-ethylcamptothecin; Partial ¹H NMR data in CDCl₃: 6 7.05 (s, 0.5H), 6.93(s, 0.5H), 5.74(d, J=16Hz, 0.5H), 5.73(d, J=16Hz, 0.5H), 5.29(d, J=16Hz, 1H), 4.41-3.75(m, 2H), 3.53-3.18(m, 2H), 2.57(q, J=6Hz, 2H), 2.26(s, 3H), 2.23(s, 3H), 2.05-1.86(m, 2H), 1.47(t, J=8Hz, 3H), 1.18-1.01(m, 3H).

### EXAMPLE 42

### Preparation of 5-(4'-aminobutoxy) camptothecin

**Step 1:** Initially 5-hydroxy camptothecin of the formula 13 where R¹=R²=R³=R⁴=R⁵=H, was prepared as described in the example 2.

**Step 2:** To a mixture of 53mg of 5-hydroxycamptothecin and 8mg of p-toluenesulfonic acid suspended in 16ml of benzene, 14mg of 4-aminobutanol was added and heated the mixture to reflux temperature for 10h. Reaction was quenched with a drop of pyridine and extracted with ethyl acetate. Organic layer was washed with water, NaHCO₃, brine and concentrated to dryness. The residue was purified by silica gel column chromatography using ethyl acetatechloroform as eluent to furnish 45mg of 5-(4-aminobutoxy) camptothecin; mp: 150°C.: IR: 3397, 1745, 1664, 1617, 1384, 1224, 1162, 1038, 684, 570 cm⁻¹; Partial ¹H NMR data in (CDCl₃+ DMSO-d6): 6 7.50(d, D₂O exchangeable, 1H), 6.95(s, 0.5H), 6.85(s, 0.5H), 6.25(d, D₂O exchangeable, 1H), 5.65(d, J=16Hz, 1H), 5.35(d, J=16Hz, 0.5H), 5.25(d, J=16Hz, 0.5H), 4.15-3.80(m, 2H), 2.15-1.65(m, 8H), 1.15-0.98(m, 3H); Mass (m/z): 436(M+1), 392, 347, 333, 305, 153, 123, 105, 90, 62;

### EXAMPLE 43

### Preparation of 5-(2'-methoxyethoxy)camptothecin

**Step 1:** Initially 5-hydroxy camptothecin of the formula 13 where R¹=R²=R³=R⁴=R⁵=H, was prepared as described in the example 2.

**Step 2:** To a mixture of 120mg of 5-hydroxycamptothecin and 0.13ml of sulfuric acid suspended in 18ml of chloroform, 20mg of ethyleneglycol monomethylether was added and heated the mixture to reflux temperature for 10h. Reaction was quenched with a drop of pyridine and extracted with ethyl acetate. Organic layer was washed with water, NaHCO₃, brine and concentrated to dryness. The residue was purified by silica gel column chromatography using ethyl acetate-chloroform as eluent to furnish 80mg of 5-(2'-methoxyethoxy)camptothecin; mp: 123°C.; IR, 3294, 2933, 1748, 1665, 1617, 1384, 1155, 1077, 1045, 761 cm⁻¹; ¹H NMR (CDCl₃) : 6 8.51(s, 1H), 8.24(d, J=8Hz, 1H), 7.93(d, J=8Hz, 1H), 7.79(t, J=6.8Hz, 1H), 7.65(t, J=6.8Hz, 1H), 7.58(s, 0.5H), 7.56(s, 0.5H), 6.91(s, 0.5H), 6.82(s, 0.5H), 5.71(d, J=16Hz, 1H), 5.28(d, J=16Hz, 1H), 4.55-4.05(m,2H), 3.95(br s, 1H, D₂O exchangeable), 3.81-3.56(m,2H), 3.48(s, 1.5H), 3.44(s, 1.5H), 1.94 (m,2H), 1.05(t, J=7Hz, 3H); Mass (m/z): 423 (M+1) , 364, 347, 319, 304, 275, 218, 128, 101; 82.

### EXAMPLE 44

### Preparation of 5-(2'N-Methylpyrrolidinoethoxy)-7-ethylcamptothecin

**Step 1:** Initially 5-hydroxy-7-ethylcamptothecin of the formula 13 where R¹=R²=R³=R⁴=H, R⁵=Et, was prepared as described in the example 4.

**Step 2:** To a mixture of 50mg of 5-hydroxy-7-ethylcamptothecin and 10mg of p-toluenesulfonic acid suspended in 15ml of benzene, 18mg of 1-methyl-2-pyrrolidinoethanol was added and heated the mixture to reflux temperature for 8h. Reaction was quenched with a drop of pyridine and extracted with ethyl acetate. Organic layer was washed with water, NaHCO₃, brine and concentrated to dryness. The residue was purified by silica gel column chromatography using methanol-chloroform as eluent to furnish 35mg of 5-(2'-N-Methylpyrrolidinoethoxy)-7-ethylcamptothecin; mp: 102°C.: Mass (m/z): 504(M+1), 460, 375, 347, 331, 275, 245, 128, 110, 84.

## Claims

1. A novel compound of the formula 1, 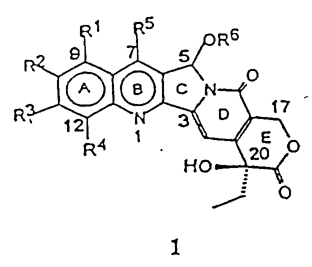 wherein R¹, R², R³ and R⁴ are independently the same or different and represent hydrogen, hydroxy, aryloxy, lower alkoxy, lower alkanoyl, nitro, cyano, halo, carboxy, carbonyloxy, amino, substituted amino, lower alkyl, substituted lower alkyl or R², R³ together represents -O-(CH₂)ₙ-O- where n=1 or 2; R⁵ represents hydrogen, lower alkyl, substituted lower alkyl, lower aralkyl, hydroxymethyl, carboxymethyl group, aminomethyl, substituted aminomethyl where the amino group is mono or disubstituted in which both substituents are independent or combined together to form 5 or 6 membered cyclic ring system containing carbon, and optionally one or two heteroatoms selected from oxygen, nitrogen or sulfur; and R⁶ represents hydrogen, phenyl or benzyl where the phenyl group may be unsubstituted or substituted with mono, di or trisubstituents which may be selected from halogen, hydroxy, lower alkoxy, cyano, carboxyl, nitro, amino or substituted amino, lower alkyl, substituted lower alkyl; cycloalkyl or cycloalkyl lower alkyl where the cyclic ring is in the range of 3 membered to 7 membered ring system containing all carbon atoms; lower alkyl groups substituted with heterocyclic rings where the heterocyclic ring system is 3 to 7 atoms containing carbons with at least one heteroatom selected from oxygen, nitrogen or sulfur; lower alkanoyl; benzoyl where the phenyl group can be unsubstituted or substituted; lower alkenyl, lower alkyl substituted lower alkyl, substituted lower alkenyl, substituted lower alkanoyl where the substituents are selected from halogen, hydroxy, alkoxy, aryloxy, thio, thioalkyl, thicaryl, aryl, heteroaryl, carboxy, cyano, nitro, amido or amino in which the amino group can be unsubstituted or mono, or disubstituted in which both substituents are independent or combined together to form 5 or 6 membered cyclic ring system containing carbon, and optionally one or more heteroatoms selected from oxygen, nitrogen or sulfur;
with the proviso that (i) when R¹ is methoxy, R⁶ is not hydrogen or lower alkyl group; (ii) when R² is hydroxy, lower alkoxy, thioalkyl, nitro, amino, alkylamino, acylamino, or halogen, R⁶ is not hydrogen or lower alkyl group; (iii) when R⁵ is lower alkyl, lower aralkyl, CH₂OH, COOH, COOMe, CH₂OR" where R" represents lower alkyl or acyl group, R⁶ is not hydrogen or lower alkyl group; (iv) when R¹ is methoxy group, R² is hydroxy, lower alkoxy, thioalkyl, nitro, amino, alkylamino, acylamino, or halogen, R⁵ is lower alkyl, lower aralkyl, CH₂OH, COOH, COOMe, CH₂OR" where R" represents lower alkyl or acyl group, R⁶ is not hydrogen or lower alkyl group; (v) when R¹ through R⁶ represent hydrogen, R⁶ is not hydrogen or lower alkyl group.

2. A compound of the formula 1, 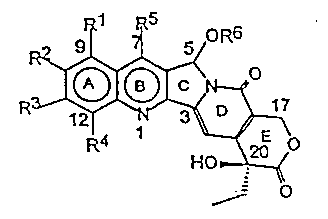 where R² represents hydroxy, R¹, R³, R⁴ and R⁵ represent hydrogen, and R⁶ represents trifluoroethyl group.

3. A compound of the formula 1, 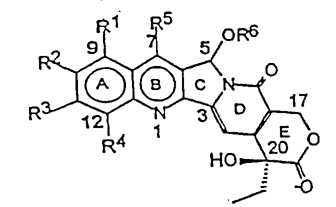 where R¹, R², R³, R⁴ and R⁵ represent hydrogen, and R⁶ represents fluoroethyl group.

4. A compound of the formula 1, 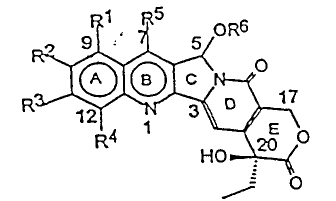 where R¹ represents N,N-dimethylaminomethyl, R² represents hydroxyl group, R³, R⁴ and R⁵ represent hydrogen, and R⁶ represents 2'-methoxyethyl group.

5. A compound of the formula 1, 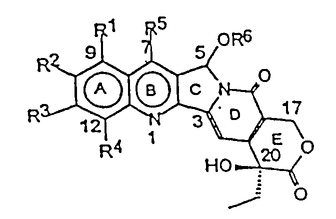 where R¹ represents nitro group, R², R³, R⁴ and R⁵ represent hydrogen, and R⁶ represents 2'-methoxyethyl group.

6. A compound of the formula 1, 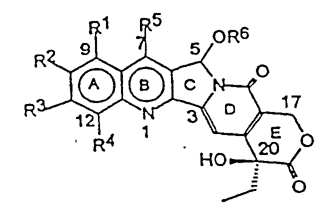 where R¹, R², R³, R⁴ and R⁵ represent hydrogen, and R⁶ represents 2'-hydroxyethyl group.

7. Compounds of the formula 1 where R¹ through R⁶ have the meaning described in any one of claims 1 to 6 as a mixture of two diastereomers, said diastereomers having 20(S),5(R) and 20(S),5(S), configurations.

8. Compounds of the formula 1 in which each of the diastereomers having 20(S),5(R) and 20(S),5(S) configurations as individual isomer, substantially free from the other isomer, where R¹ through R⁶ have the meaning described in claims 1 to 6.

9. A pharmaceutical composition comprising compounds of the formula 1 as defined in any one of claims 1 to 6 or a derivative thereof and a pharmaceutically acceptable non-toxic excipient, diluent or solvent.

10. The use of the novel compounds of the formula 1 as defined in any one of claims 1 to 9 for the treatment of cancers, leukemia and HIV related conditions.

11. A medicine for treating cancer, leukemia or HIV related conditions comprising a compound according to any one of claims 1 to 9 or a derivative thereof and a pharmaceutically acceptable, carrier, diluent or solvent.

12. Use of a compound according to any one of claims 1 to 9 for manufacture of a medicament for treatment of cancer, leukemia or HIV related conditions.

13. A process for the preparation of novel compounds of the formula 1, which comprises,
(i) reacting the compounds of the formula 2, 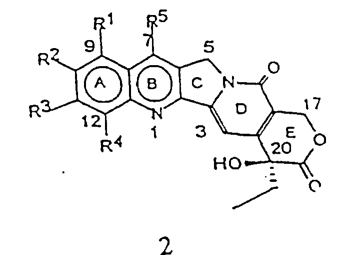 where R¹ to R⁵ have the meaning described in claim 1, in the presence of acid and a ferric salt, with a compound having the formula R⁶-OH where R⁶ represents lower alkyl lower alkenyl, (C₃-C₇)cycloalkyl, haloalkyl or hydroxyalkyl to obtain compounds of the formula 12 and compounds of the formula 13, 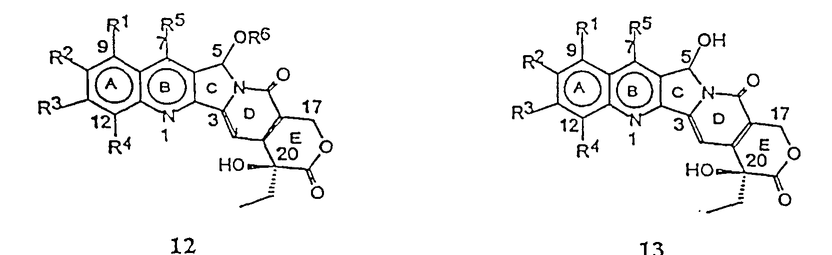 wherein R¹, R², R³, R⁴ and R⁵ have the meaning given in claim 1,
(ii) separating the compounds of the formulae 12 and 13 prepared in the step (i),
(iii) hydrolyzing the compounds of the formula 12 to obtain additional amounts of the compounds of the formula 13,
(iv) reacting the compound of the formula 13, in the presence of an acid, with a compound having the formula R6-OH to obtain compounds of the formula 1, 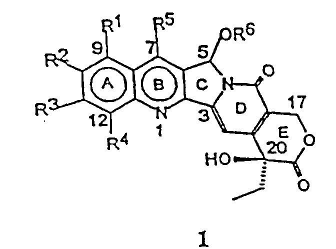 where R¹, R², R³, R⁴ and R⁵ have the meaning described in claim 1 and R⁶ is as defined in claim 1.

14. A process for the preparation of a compound of formula 1, where R¹, R³, R⁴ and R⁵ are hydrogen, R² represents hydroxyl group and R⁶ represents trifluoroethyl group, which comprises,
(i) reacting the compound of the formula 2, 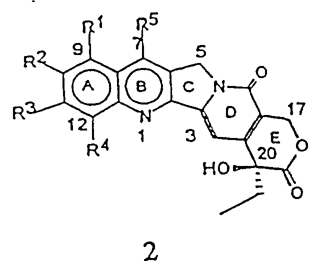 where R¹, R³, R⁴ and R⁵ are hydrogen, R² represents hydroxyl group, in the presence of conc.sulfuric acid and ferric chloride trihydrate with ethanol and heating the mixture to reflux conditions to obtain compound of the formula 12 and compound of the formula 13, 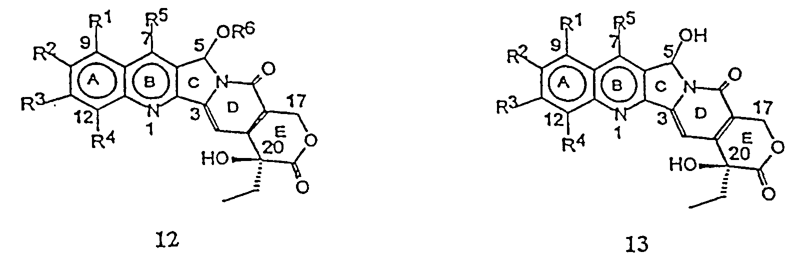 wherein R¹, R³, R⁴ and R⁵ represent hydrogen, R² is hydroxyl group and R⁶ represents ethyl group,
(ii) separating the compounds of the formulae 12 and 13 prepared in the step (i),
(iii) hydrolyzing the compounds of the formula 12, by dissolving in aqueous ethanol and refluxing with hydrochloric acid, to obtain additional amounts of the compound of the formula 13,
(iv) reacting the compound of the formula 13, in the presence of conc.sulfuric acid, with trifluoroethanol dissolved in dichloroethane solvent, to obtain the compound of the formula 1, 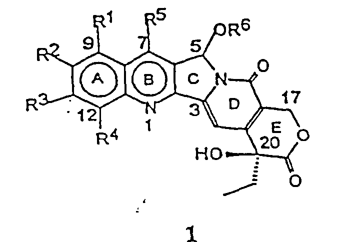 where R¹, R³, R⁴ and R⁵ are hydrogen, R² represents hydroxyl group and R⁶ represents trifluoroethyl group.

15. A process for the preparation of compounds of formula 1, 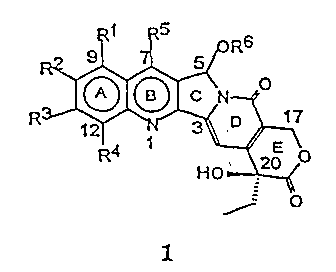 wherein R⁶ represents hydrogen or lower alkyl, R¹ represents hydrogen or methoxy, R² represents hydrogen, hydroxy, lower alkoxy, acyloxy, SH, thioalkyl, thioacyl, nitro, amino, alkylamino, acylamino or halogen; R³ and R⁴ are hydrogen and R⁵ represents hydrogen, lower alkyl, lower aralkyl, CH₂OH, COOH, COOMe or CH₂OR' where R' represents lower alkyl or acyl group which comprises,
(i) reacting the compounds of the formula 2, 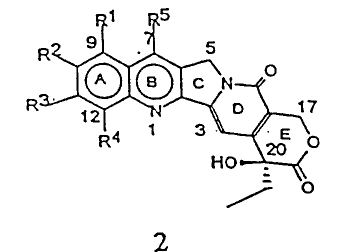 where R¹ to R⁵ have the meaning described above, in the presence of an acid and ferric salt, with a compound of the formula R⁶-OH where R⁶ represents lower alkyl groups, to obtain compounds of the formula 12 and compounds of the formula 13, 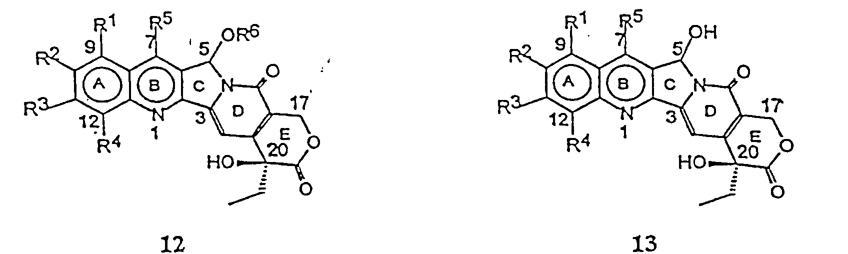 wherein R¹, R², R³, R⁴ and R⁵ have the meaning given above,
(ii) separating the compounds of the formulae 12 and 13 prepared in the step (i),
(iii) hydrolyzing the compounds of the formula 12 to obtain additional amounts of the compounds of the formula 13.
(iv) reacting the compound of the formula 13, in the presence of an acid, with a compound having the formula R⁶-OH to obtain compounds of the formula 1, 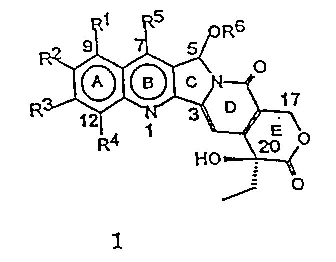 where R¹, R², R³, R⁴, R⁵ and R⁶ have the meaning described above.

16. The process according to claim 13, wherein the ferric salt is ferric chloride.

17. The process according to claim 15, wherein the ferric salt is ferric chloride.
